# FASCICULE DE BREVET EUROPEEN

(11) **EP 3 405 217 B1**
(45) Date de publication et mention de la délivrance du brevet: **03.06.2020**
(21) Numéro de dépôt: 17703928.6
(22) Date de dépôt: 18.01.2017
(51) Int. Cl.: A61K 49/00, A61K 41/00

(54) **NANOPARTICULES D'ORGANOSILICE MESOPOREUSES, LEUR METHODE DE PREPARATION ET LEURS UTILISATIONS.**
NANOPARTIKEL VON MESOPORÖSEN ORGANOSILICIUM, IHRE HERSTELLUNGSMETHODE UND IHRE VERWENDUNGEN
NOVEL NANOPARTICLES OF MESOPOROUS ORGANOSILICIUM, THEIR MANUFACTURING METHOD AND THEIR USES

(30) Priorité: 19.01.2016 FR 1650396
(43) Date de publication de la demande: 28.11.2018
(73) Titulaire: Centre National de la Recherche Scientifique (CNRS), 75016 Paris (FR); Université de Montpellier, 34090 Montpellier (FR); Nanomedsyn, 34090 Montpellier (FR)
(72) Inventeur: DURAND, Jean-Olivier, 34250 Palavas-les-flots (FR); MAURIELLO JIMENEZ, Chiara, 34000 Montpellier (FR); RICHETER, Sébastien, 34090 Montpellier (FR); RAEHM, Laurence, 34080 Montpellier (FR); GARY-BOBO, Magali, 34170 Castelnau-le-Lez (FR); GARCIA, Marcel, 34730 Prades-le-Lez (FR); MAYNADIER, Marie, 34800 Ceyras (FR)
(74) Mandataire: Lavoix
(86) Numéro de dépôt international: PCT/EP2017/050946
(87) Numéro de publication internationale: WO 2017/125413

(56) Documents cités:
- WO-A1-2014/000180
- FR-A1- 2 935 974
- FR-A1- 2 988 721
- KOICHIRO HAYASHI ET AL: "Photostable Iodinated Silica/Porphyrin Hybrid Nanoparticles with Heavy-Atom Effect for Wide-Field Photodynamic/Photothermal Therapy Using Single Light Source", ADVANCED FUNCTIONAL MATERIALS, vol. 24, no. 4, 1 janvier 2014 (2014-01-01), pages 503-513, XP055303302, DE ISSN: 1616-301X, DOI: 10.1002/adfm.201301771
- OUAHIBA HOCINE ET AL: "Silicalites and Mesoporous Silica Nanoparticles for photodynamic therapy", INTERNATIONAL JOURNAL OF PHARMACEUTICS, vol. 402, no. 1-2, 15 décembre 2010 (2010-12-15), pages 221-230, XP055039772, ISSN: 0378-5173, DOI: 10.1016/j.ijpharm.2010.10.004
- MAGALI GARY-BOBO ET AL: "Cancer therapy improvement with mesoporous silica nanoparticles combining targeting, drug delivery and PDT", INTERNATIONAL JOURNAL OF PHARMACEUTICS, vol. 423, no. 2, 1 février 2012 (2012-02-01), pages 509-515, XP055232901, NL ISSN: 0378-5173, DOI: 10.1016/j.ijpharm.2011.11.045
- CHIARA MAURIELLO-JIMENEZ ET AL: "Porphyrin-functionalized mesoporous organosilica nanoparticles for two-photon imaging of cancer cells and drug delivery", JOURNAL OF MATERIALS CHEMISTRY B, vol. 3, no. 18, 1 janvier 2015 (2015-01-01), pages 3681-3684, XP055302691, GB ISSN: 2050-750X, DOI: 10.1039/C5TB00315F
- YU CHEN ET AL: "Hollow Mesoporous Organosilica Nanoparticles: A Generic Intelligent Framework-Hybridization Approach for Biomedicine", JOURNAL OF THE AMERICAN CHEMICAL SOCIETY, vol. 136, no. 46, 19 novembre 2014 (2014-11-19), pages 16326-16334, XP055303446, US ISSN: 0002-7863, DOI: 10.1021/ja508721y

## Description

La présente invention concerne des nanoparticules d'organosilice mésoporeuses, leur méthode de préparation et leurs utilisations dans le traitement par thérapie photodynamique ou dans l'imagerie.

La thérapie photodynamique est une méthode thérapeutique non-invasive et sélective permettant de détruire les cellules cancéreuses ou infectées à l'aide d'un agent photosensibilisant susceptible d'être activé par une source lumineuse de longueur d'onde appropriée, dans le domaine du visible ou du proche infra-rouge. Par action combinée d'un agent photosensibilisant et de lumière, l'oxygène moléculaire à l'état triplet naturel (³O₂) est converti en oxygène singulet (¹O₂) hautement cytotoxique et en autres espèces oxydantes réactives (O²·-, OH·). L'oxygène singulet ¹O₂ est un puissant oxydant qui réagit avec de nombreux constituants cellulaires tels les triacyles glycérols saturés, le cholestérol des membranes, les phospholipides, les acides aminés (histidine, tryptophane, méthionine) et les acides nucléiques. L'oxygène singulet peut par conséquent conduire à la destruction des cellules cancéreuses qui sont en contact avec lui mais est également toxique pour les cellules qui les entourent. En l'absence d'exposition à la source lumineuse appropriée, l'agent photosensibilisant n'est pas toxique pour les cellules, ce qui permet de réduire les effets secondaires par rapport à la chimiothérapie ou la radiothérapie.

La plupart des agents photosensibilisants connus dans l'art antérieur sont excités par absorption d'un photon. Dans ce mode d'excitation, l'agent photosensibilisant est activé tout le long du trajet du rayon laser. Comme la radiation ne peut pas se localiser spécifiquement aux cellules à traiter, le risque d'endommager les tissus superposés ou adjacents aux tissus à traiter est l'un des désavantages de la thérapie photodynamique via l'excitation d'un photon.

Par ailleurs, la plupart des agents photosensibilisants développés à ce jour absorbent une lumière dans le domaine du visible qui ne peut pénétrer dans la peau qu'à une profondeur de quelques millimètres, ce qui limite considérablement le domaine d'application de la thérapie photodymanique.

La thérapie photodynamique par l'excitation de deux photons s'est développée dans les dernières années et a pour objectif de surmonter ces inconvénients techniques.

Dans le mode d'excitation par deux photons, un agent photosensibilisant est excité simultanément par l'absorption de deux photons pour passer de son état électronique de repos à un état excité. Dans ce mode d'excitation, ledit agent est activé uniquement au focus des rayons laser. La radiation peut par conséquent être localisée plus précisément aux tissus à traiter. De plus, ce mode d'excitation est obtenu par une lumière proche infra-rouge, qui peut pénétrer plus profondément dans les tissus.

L'excitation par deux photons peut être obtenue par une approche indirecte à l'aide de la méthode FRET (transfert d'énergie entre molécules fluorescentes ou transfert d'énergie par résonance de type Förster) ou une approche directe en utilisant un agent photosensibilisant capable d'absorber deux photons.

La plupart des agents photosensibilisants absorbant deux photons utilisés à ce jour sont des molécules organiques. L'utilisation de ces molécules organiques photosensibilisantes est toutefois soumise aux limites suivantes : ces molécules sont hydrophobes et nécessitent un système de délivrance, notamment une formulation particulière, en particulier sous forme de nanoparticules, pour une application clinique. Ces molécules ne ciblent pas efficacement les cellules tumorales et présentent un temps de résidence important dans les tissus sains. Etant donné que la plupart de ces molécules sont activées par la lumière visible, cela engendre des effets secondaires de destruction des cellules saines et ces molécules ne peuvent pas être utilisées pour le traitement des tissus plus profonds.

La demande internationale WO 2013/144154 décrit des nanoparticules de silice poreuses sur lesquelles sont greffés des dérivés de porphyrine. Cependant, ces nanoparticules obtenues après greffage ne sont pas assez sensibles à une excitation biphotonique pour une application dans la thérapie photodynamique par excitation à deux photons.

Une autre approche prometteuse développée dans les dernières années consiste à intégrer les agents photosensibilisants dans des nanoparticules de silices, notamment les nanoparticules d'organosilice mésoporeuses.

Les nanoparticules d'organosilice mésoporeuses (PMO) périodiques appartiennent à une nouvelle classe de matériaux mésoporeux et suscitent de plus en plus d'intérêt pour ses applications dans l'adsorption de molécules ou gaz, la catalyse, l'immobilisation d'enzymes, et notamment la délivrance de médicament, en raison de leur biocompatibilité, de leur faible activité hémolytique et leur surface spécifique très élevée. Ces nanoparticules PMO sont préparées à partir des alcoxysilanes qui sont connectés par des groupements organiques pouvant conférer aux nanoparticules différentes propriétés chimiques.

Mauriello-Jimenez et al. ont décrit un type de nanoparticules d'organosilice mésoporeuses comportant un dérivé de porphyrine métallé au Zn ayant huit groupes triéthoxysilyles (J. Mater. Chem. B, 2015, 3, 3681-3684). Ces nanoparticules sont capables d'émettre une fluorescence après une excitation par deux photons et peuvent par conséquent être appliquées dans l'imagerie de fluorescence à deux photons. Cependant, elles ne peuvent pas être utilisées dans la thérapie photodynamique, en raison d'un défaut de capacité à générer l'oxygène singulet après excitation biphotonique pour détruire les cellules cancéreuses.

Hayashi et al. (Adv. Funct. Mater. 2014, 24, 503-513) décrivent des nanoparticules hybrides formées par des dérivés de porphyrines et des silices iodés. Cependant, étant donné que la méthode de préparation décrite dans ce document n'utilise pas de tensioactif permettant d'induire la porosité, les nanoparticules obtenues ne sont pas poreuses.

WO 2014/000180 décrit des structures de silice mésoporeuse obtenues par la réaction entre un agent de couplage silane et un dérivé de porphyrine. Etant donné que le précureur utilisé dans ce document est le tetraéthoxysilane ou le tétraméthyxoysilane, les nanoparticules obtenues sont des nanoparticules mésoporeuse de silice (MSN), mais ne sont pas des nanoparticules d'oragnosilice mésoporeuse (PMO).

Chen et al. (J. Am. Chem. Soc. 2014, 136, 10326-16334) décrivent des nanoparticules d'organosilice mésoporeuses formant une cage moléculaire permettant d'encapsuler la doxorubicine. Ces nanoparticules ne sont pas photosensibles et ne peuvent pas être utilisées comme agent photosensibilisant dans la thérapie photodynamique.

Il y a donc toujours une nécessité de développer de nouveaux agents photosensibilisants pouvant être utilisés dans la thérapie photodynamique, notamment capables d'être excités par deux photons.

Contre toute attente, les Inventeurs de la présente invention ont trouvé une nouvelle classe de nanoparticules d'organosilice mésoporeuses contenant des dérivés de porphyrine, lesdites nanoparticules présentant une augmentation importante de la section efficace d'absorption à deux photons, et donc capables de générer l'oxygène singulet après l'irradiation par une lumière proche de l'infrarouge, cela permettant de détruire les cellules cancéreuses.

Le premier aspect de la présente invention a pour objet de proposer de nouvelles nanoparticules d'organosilice mésoporeuses contenant des dérivés de porphyrine.

Un autre objet de l'invention concerne des compositions pharmaceutiques comprenant lesdites nanoparticules d'organosilice mésoporeuses, notamment des compositions pharmaceutiques pour leur utilisation dans le traitement de cancers, notamment par la thérapie photodynamique à excitation par un photon ou par deux photons.

Un autre objet de l'invention concerne l'utilisation desdites nanoparticules en tant que photosensibilisants.

L'invention concerne également l'utilisation desdites nanoparticules dans l'imagerie de fluorescence à un photon ou à deux photons.

L'invention a également pour objet une méthode de préparation desdites nanoparticules, ainsi que les nanoparticules obtenues par ce procédé.

La présente invention propose également un kit de détection d'une pathologie, telle qu'un cancer, une tumeur, ou une maladie proliférative cellulaire.

Les nanoparticules d'organosilice mésoporeuses de la présente invention sont formées par des éléments comprenant ou constitués par :
(i) un dérivé de porphyrine choisi parmi :
   - un composé de formule A 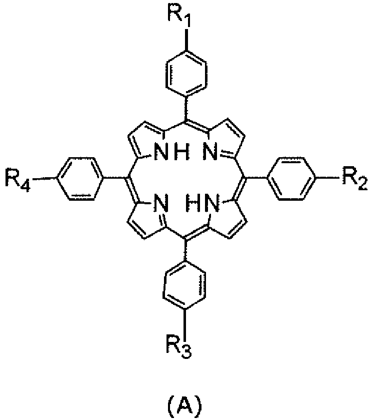 dans laquelle :
      - Soit R₁, R₂, R₃, et R₄ correspondent tous à 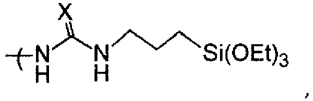 où X est l'atome d'oxygène ou l'atome de soufre,
      - Soit R₁, R₂, R₃, et R₄ correspondent tous à 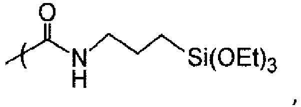
   - un composé de formule B, C ou D, dans lesquelles Z est choisi parmi l'atome d'oxygène ou l'atome de soufre 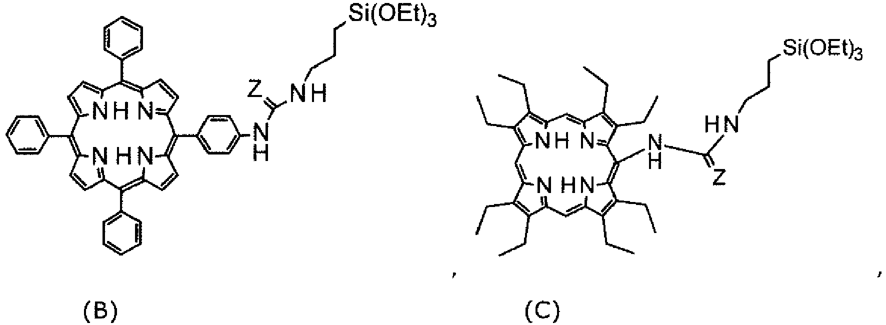 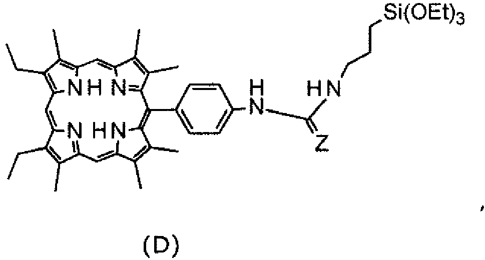 ou
   - un composé E, F ou G 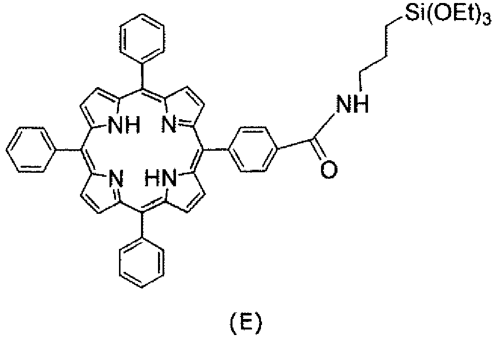 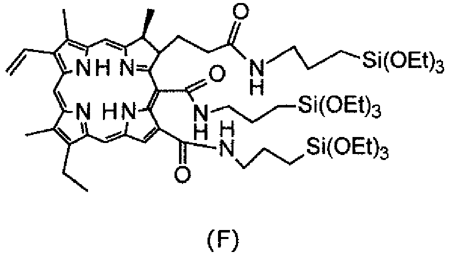 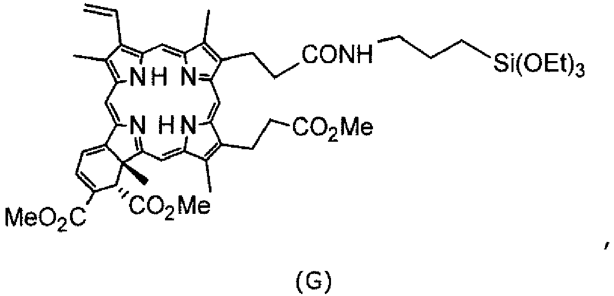 et
(ii) un composé de formule I :

   (EtO)₃Si(CH₂)ₙSi(OEt)₃ (I),

   dans laquelle n représente un nombre entier choisi entre 1 et 10, et éventuellement
(iii) un composé de formule II

   (EtO)₃Si(CH₂)₃-(S-S)ₘ-(CH₂)₃-Si(OEt)₃ (II)

   dans laquelle m est un entier égal à 2 ou 4,
lesdites nanoparticules éventuellement encapsulant au moins un composé anticancéreux hydrophile et/ou hydrophobe.

On entend par « nanoparticules d'organosilice mésoporeuses » une classe de nanoparticules poreuses obtenues du précurseur organotriethoxysilane, dont la taille de pores est généralement de 2 à 50 nm, dans lesquelles les groupes organiques sont intégrés de manière covalente dans la matrice des particules et forment la structure des particules.

Les nanoparticules de la présente invention incorporent de manière covalente des porphyrines tétrasilylées dans la matrice et possèdent des pores de taille de 2 à 10 nm, notamment d'environ 3 nm.

Un dérivé de porphyrine, un composé de formule I et éventuellement un composé de formule II tels que définis ci-dessus sont les éléments structurels permettant de former la matrice des nanoparticules d'organosilice mésoporeuses de base par une co-condensation aux conditions appropriées.

Les nanoparticules de base sont photosensibilisantes, et peuvent être activées notamment par une source lumineuse en mono- ou biphotonique, en particulier par un rayonnement ayant une longueur d'onde de 400-1000 nm, notamment un rayonnement ayant une longueur d'onde d'environ 800 nm, et capable de générer l'oxygène singulet.

En bi-photonique, l'excitation peut être obtenue par un rayonnement ayant une longueur d'onde de 750 à 1000 nm, notamment de 800 nm, et est faite préférentiellement par trois à douze scans de 1,57 s chacun à une puissance de 20 à 140 mW, avantageusement de 50 à 100mW, et encore mieux aux environs de 80 mW et pour une surface scannée de 1,5×1,5 mm², soit une fluence de 10,6 J/cm² à 42,4 J/cm² par microscopie multiphotonique. En monophotonique, l'irradiation peut être obtenue à une longueur d'onde de 400 à 700 nm, notamment à 405 nm, et est faite préférentiellement par irradiation pendant 10 min à une puissance de 10 mW/0,32 cm² soit une fluence de 18,75 J/cm².

Dans le cadre de la présente invention, les termes "excitation", "activation" et "irradiation" sont interchangeables lorsqu'ils concernent l'absorption d'un ou deux photons par les nanoparticules de l'invention.

Les termes "bi-photonique" et "à deux photons" peuvent être remplacés l'un par l'autre.

Les termes "mono-photonique" et "à un photon" sont également interchangeables.

Le diamètre des nanoparticules d'organosilice mésoporeuses de base décrites dans la présente invention peut être de 20 à 400 nm, avantageusement de 50 à 300 nm, plus avantageusement de 200-250 nm. Ces nanoparticules possèdent une surface spécifique très élevée, pouvant être de 100 à 1500 m²/g, avantageusement de 800-1000 m²/g. Ceci confère aux nanoparticules de l'invention une efficacité très élevée pour absorber des molécules de petite taille. Grâce à cette propriété, les nanoparticules de l'invention peuvent encapsuler efficacement au moins un type de molécules chimiques ayant une masse moléculaire inférieure à 1000 Dalton, notamment un médicament de petite taille, tel qu'un composé anticancéreux hydrophile et/ou hydrophobe.

Conformément à l'invention, le composé anticancéreux hydrophile peut être choisi parmi la gemcitabine, la gemcitabine monophosphate, le 5-fluorouracile, la cytarabine, le topotécane, l'irinotécane, ou l'oxalylplatine ; le composé anticancéreux hydrophobe peut être choisi parmi la doxorubicine, le paclitaxel, ou la camptothécine.

Contrairement aux nanoparticules de silice mésoporeuses connues dans l'art antérieur, qui ne peuvent pas encapsuler efficacement un composé hydrophile, tel que la gemcitabine ou la gemcitabine monophosphate, les nanoparticules de base de l'invention, grâce à l'environnement hydrophobe à l'intérieur de ses pores, sont particulièrement efficaces et avantageuses pour encapsuler les composés anticancéreux hydrophiles, notamment pour encapsuler la gemcitabine ou la gemcitabine monophosphate.

Les nanoparticules de base de l'invention sont également aptes à encapsuler plusieurs types de molécules, par exemple un composé hydrophile et un composé hydrophobe.

Dans un mode de réalisation particulier, les nanoparticules d'organosilice mésoporeuses de l'invention sont formées par un dérivé de porphyrine de formule A tel que défini ci-dessus. Lesdites porphyrines s'agrègent en agrégat J avec un décalage du spectre UV-Vis vers le rouge. Ceci confère aux nanoparticules la propriété biphotonique avec une augmentation importante de la section efficace d'absorption à deux photons et peuvent être excitées par une lumière ayant une longueur d'onde de 750 nm à 800 nm.

On entend par « propriété biphotonique » la capacité pour les nanoparticules d'absorber simultanément deux photons.

On entend par « agrégat J » un type de colorants ayant une bande d'absorption décalée vers une longueur d'ondes plus élevée avec un plus grand coefficient d'absorption lorsqu'il s'agrège par auto-organisation en supramolécule sous influence d'un solvant ou additif.

Les nanoparticules d'organosilice mésoporeuse de la présente invention dans lesquelles sont intégrées les dérivés de porphyrine de formule A sont photosensibles à l'excitation bi-photonique ou mono-photonique et peuvent générer l'oxygène singulet après l'excitation, ce qui permet à ce type de nanoparticules d'être utilisé dans la thérapie photodynamique, notamment la thérapie photodynamique par excitation à deux photons.

Dans un mode de réalisation, les nanoparticules de la présente invention sont formées par les éléments comprenant un dérivé de porphyrine correspondant à la formule A1: 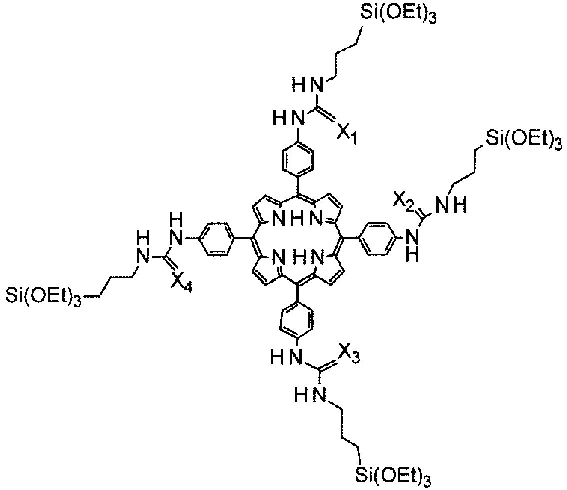 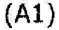 dans laquelle X₁, X₂, X₃, et X₄ sont choisis indépendamment l'un de l'autre parmi l'atome d'oxygène ou l'atome de soufre.

Un mode de réalisation de l'invention concerne les nanoparticules d'organosilice mésoporeuses formées par les éléments constitués par:
(i) un dérivé de porphyrine tel défini ci-dessus, et
(ii) un composé de formule I tel que défini ci-dessus.

Dans le cadre de la présente invention, un composé de formule I peut être notamment le bis(triéthoxysilyl)méthane, le bis(triéthoxysilyl)éthane, le 1,3-bis(triéthoxysilyl)propane, le 1,4-bis(triéthoxysilyl)butane, ou le 1,5-bis(triéthoxysilyl)pentane.

Le dérivé de porphyrine et le composé de formule I tels que définis ci-dessus peuvent être présents dans les nanoparticules de l'invention avec un rapport en mole entre 2 : 98 et 20 : 80, notamment 10 : 90.

Dans un mode de réalisation particulier, les nanoparticules sont formées par les éléments constitués par
(i) un dérivé de porphyrine de formule A, notamment un dérivé de formule A1a 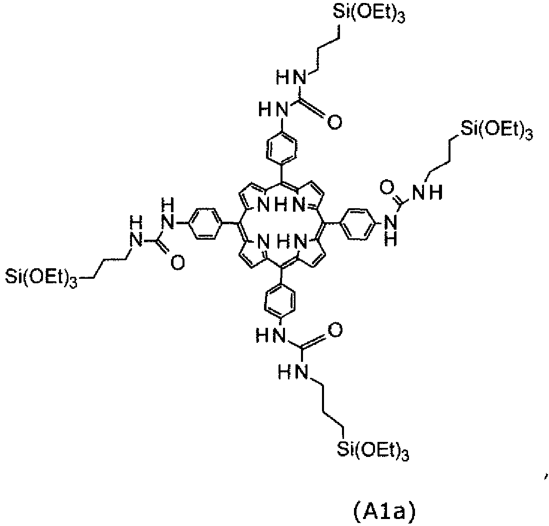 et
(ii) un composé de formule I tel que défini ci-dessus.

Ces nanoparticules sont capables d'être excitées par deux photons dans le domaine infra-rouge et de générer l'oxygène singulet.

Conformément à l'invention, on entend par les termes « dans le domaine infra-rouge », « proche de l'infra-rouge » la longueur d'onde d'une source lumineuse comprise entre 700 et 1000 nm.

Un mode de réalisation plus particulier de l'invention concerne les nanoparticules, désignées ci-après "NPs CM238", formées par les éléments constitués par :
(i) un dérivé de porphyrine de formule Ala, et
(ii) le bis(triéthoxysilyl)éthane.

Dans un autre mode de réalisation particulier, les nanoparticules sont formées par les éléments constitués par
(i) un dérivé de porphyrine de formule B, et
(ii) un composé de formule I tel que défini ci-dessus.

Un autre mode de réalisation plus particulier de l'invention concerne les nanoparticules, désignées ci-après "NPs PMOS1", formées par les éléments constitués par :
(i) un dérivé de porphyrine de formule B, et
(ii) le bis(triéthoxysilyl)éthane.

Un mode de réalisation de l'invention concerne les nanoparticules d'organosilice mésoporeuses de base formées par les éléments constitués par:
(i) un dérivé de porphyrine tel défini ci-dessus,
(ii) un composé de formule I tel que défini ci-dessus, et
(iii) un composé de formule II tel que défini ci-dessus.

Grâce à la présence d'un composé de formule II, ce type des nanoparticules de base est biodégradable dans un délai de 48H en se dégradant en composés éliminés par clairance rénale avec une absence de toxicité.

Conformément à l'invention, un composé de formule II est notamment le bis triéthoxysilylpropyl disulfure.

Au sein de la matrice de ce type de nanoparticules de l'invention, la proportion en mole entre un dérivé de porphyrine, un composé de formule I et un composé de formule II peut être respectivement de 1 à 30%, de 10 à 90% et de 10 à 90%.

Avantageusement dans les nanoparticules constituées par un dérivé de porphyrine, un composé de formule I et un composé de formule II, la proportion en mole est respectivement de 1 à 30% pour le dérivé de porphyrine, de 40% à 90% pour le composé de formule I, inférieure à 50% pour le composé de formule II.

Dans un mode de réalisation particulier, les nanoparticules sont formées par les éléments constitués par
(i) un dérivé de porphyrine de formule A, notamment un dérivé de formule A1a tel que défini ci-dessus,
(ii) un composé de formule I tel que défini ci-dessus,
(iii) un composé de formule II tel que défini ci-dessus.

Ces nanoparticules sont à la fois biodégradables et capables d'être excitées par deux photons dans le domaine infra-rouge.

Un mode de réalisation plus particulier de l'invention concerne les nanoparticules, désignées ci-après "NPs CM240", formées par les éléments constitués par :
(i) un dérivé de porphyrine de formule A1a,
(ii) le bis(triéthoxysilyl)éthane,
(iii) le bis triéthoxysilylpropyl disulfure.

Plus particulièrement, l'invention concerne les nanoparticules, désignées ci-après « NP CM240-b », formées par les éléments constitués par :
(i) un dérivé de porphyrine de formule A1a dont la proportion en mole est de 1 à 10%,
(ii) le bis(triéthoxysilyl)éthane dont la proportion en mole est de 40 à 90%,
(iii) le bis triéthoxysilylpropyl disulfure dont la proportion en mole est inférieure à 50%.

Un autre mode de réalisation de l'invention concerne les nanoparticules d'organosilice mésoporeuses dont la matrice est formée par un dérivé de porphyrine tel que défini ci-dessus, un composé de formule I tel que défini ci-dessus, et éventuellement un composé de formule II tel que défini ci-dessus, encapsulant au moins un composé anticancéreux hydrophile et/ou hydrophobe, notamment un composé anticancéreux hydrophile.

Ce type des nanoparticules montre une activité anticancéreuse à la fois par la production de l'oxygène singulet après l'excitation des nanoparticules par une source lumineuse proche de l'infra-rouge et l'activité du composé anticancéreux absorbé au sein des nanoparticules et montre un effet synergique par rapport à l'addition de l'activité anticancéreuse obtenue par ledit composé anticancéreux seul et de celle obtenue par la thérapie photodynamique seule.

Lorsqu'un composé anticancéreux hydrophile ou hydrophobe est présent dans les nanoparticules de l'invention, la charge en composé anticancéreux hydrophile ou hydrophobe tel que défini ci-dessus est de 2% à 100% en masse, notamment de 40% en masse, par rapport à la masse initiale des nanoparticules avant l'encapsulation dudit composé anticancéreux.

Dans un mode de réalisation particulier, les nanoparticules sont formées par les éléments constitués par:
(i) un dérivé de porphyrine tel que défini ci-dessus,
(ii) un composé de formule I tel que défini ci-dessus,
lesdites nanoparticules encapsulant au moins un composé anticancéreux hydrophile et/ou hydrophobe.

Dans un mode de réalisation particulier de l'invention, les nanoparticules sont formées par les éléments constitués par :
(i) un dérivé de porphyrine de formule A, notamment un dérivé de formule A1a décrite ci-dessus,
(ii) un composé de formule I tel que défini ci-dessus,
lesdites nanoparticules encapsulant la gemcitabine ou la gemcitabine monophosphate.

Un mode de réalisation plus particulier de l'invention concerne les nanoparticules, désignées ci-après "NPs CM238+gemcitabine", formées par les éléments constitués par :
(i) un dérivé de porphyrine de formule A1a,
(ii) le bis(triéthoxysilyl)éthane,
lesdites nanoparticules encapsulant la gemcitabine.

Un autre mode de réalisation de l'invention concerne les nanoparticules formées par les éléments constitués par:
(i) un dérivé de porphyrine tel défini ci-dessus,
(ii) un composé de formule I tel que défini ci-dessus,
(iii) un composé de formule II tel que défini ci-dessus,
lesdites nanoparticules encapsulant au moins un composé anticancéreux hydrophile et/ou hydrophobe.

Ce type de nanoparticules sont biodégradables et montrent une activité anticancéreuse synergique par rapport à l'addition de l'activité anticancéreuse obtenue par ledit composé anticancéreux seul et celle obtenue par la thérapie photodynamique seule.

Dans un mode de réalisation particulier de l'invention, les nanoparticules sont formées par les éléments constitués par :
(i) un dérivé de porphyrine de formule A, notamment un dérivé de formule A1a décrite ci-dessus,
(ii) un composé de formule I tel que défini ci-dessus,
(iii) un composé de formule II tel que défini ci-dessus,
lesdites nanoparticules encapsulant la gemcitabine ou la gemcitabine monophosphate.

Un mode de réalisation plus particulier de l'invention concerne les nanoparticules, désignées ci-après "NPs CM240+gemcitabine", formées par les éléments constitués par :
(i) un dérivé de porphyrine de formule A1a,
(ii) le bis(triéthoxysilyl)éthane,
(iii) le bis triéthoxysilylpropyl disulfure,
lesdies nanoparticules encapsulant la gemcitabine.

Un mode de réalisation encore plus particulier de l'invention concerne les nanoparticules, désignées ci-après « NPs CM240-b+gemcitabine », formées par les éléments constitués par :
i) un dérivé de porphyrine de formule A1a,
(ii) le bis(triéthoxysilyl)éthane,
(iii) le bis triéthoxysilylpropyl disulfure,
la proportion en mole entre le porphyrine, le bis(triéthoxysilyl)éthane et le bis triéthoxysilylpropyl disulfure étant respectivement de 1 à 30% pour le dérivé de porphyrine, de 40% à 90% pour le bis(triéthoxysilyl)éthane, inférieure à 50% pour le bis triéthoxysilylpropyl disulfure,
lesdies nanoparticules encapsulant la gemcitabine.

Un autre mode de réalisation particulier de l'invention concerne les nanoparticules formées par les éléments constitués par :
(i) un dérivé de porphyrine de formule B,
(ii) un composé de formule I tel que défini ci-dessus,
(iii) un composé de formule II tel que défini ci-dessus,
lesdites nanoparticules encapsulant la gemcitabine ou la gemcitabine monophosphate.

Dans un mode de réalisation plus particulier de l'invention, les nanoparticules, désignées ci-après « PMOS1+gemcitabine », formées par les éléments constitués par :
(i) un dérivé de porphyrine de formule B,
(ii) le bis(triéthoxysilyl)éthane
(iii) le bis triéthoxysilylpropyl disulfure
lesdies nanoparticules encapsulant la gemcitabine.

La présente invention a également pour objet de proposer un procédé de préparation des nanoparticules telles que décrites ci-dessus.

Ledit procédé comprend les étapes consistant à:
(a) faire réagir en solution aqueuse à une température de 20°C à 50°C en présence d'un tensioactif, les composés comprenant :
   (i) un dérivé de porphyrine choisi parmi :
      - un composé de formule A 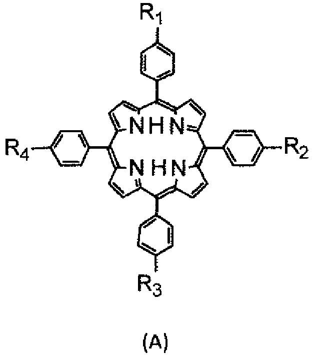 dans laquelle :
         - Soit R₁, R₂, R₃, et R₄ correspondent tous à 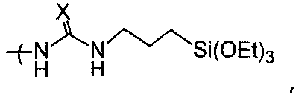 où X est l'atome d'oxygène ou l'atome de soufre,
         - Soit R₁, R₂, R₃, et R₄ correspondent tous à 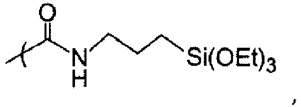
      - un composé de formule B, C ou D, dans lesquelles Z est choisi parmi l'atome d'oxygène ou l'atome de soufre 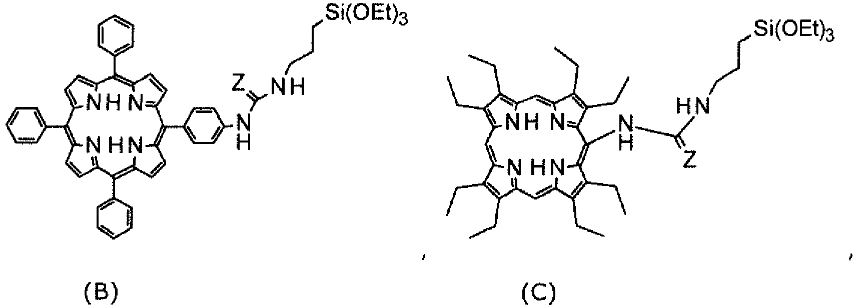 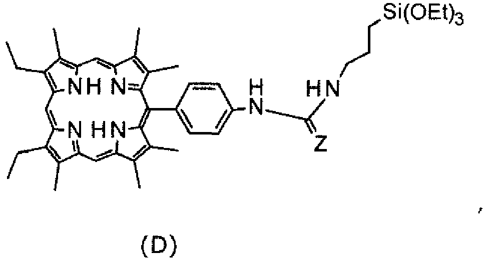 ou
      - un composé E, F ou G 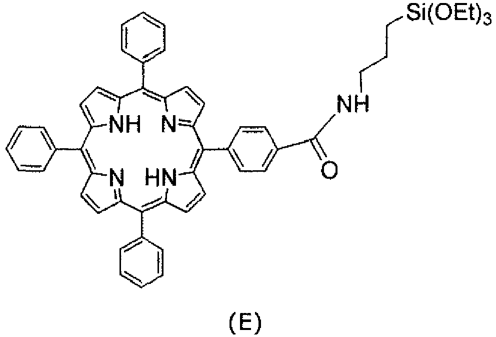 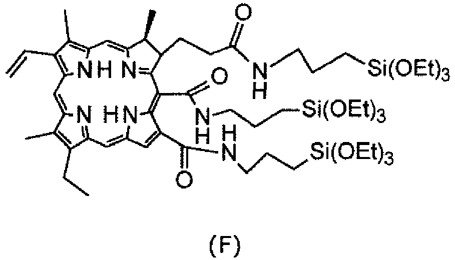 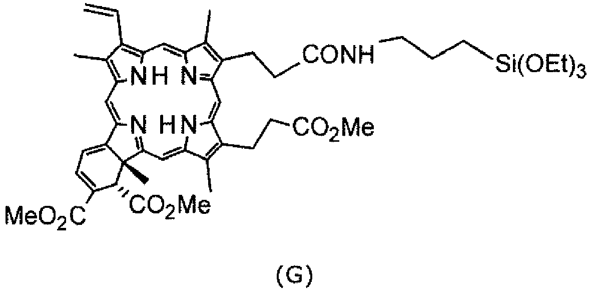
   (ii) un composé de formule I ci-après

      (EtO)₃Si(CH₂)ₙSi(OEt)₃,

      dans laquelle n représente un entier choisi entre 1 et 10, et éventuellement
   (iii) un composé de formule II

      (EtO)₃Si(CH₂)₃-(S-S)ₘ-(CH₂)₃-Si(OEt)₃

      dans laquelle m est un nombre entier égal à 2 ou 4, et
(b) récupérer les nanoparticules formées dans l'étape précédente, et éventuellement,
(c) faire réagir dans un solvant, tel que H₂O, DMSO..., les nanoparticules obtenues dans l'étape (b) avec au moins un composé anticancéreux hydrophile et/ou hydrophobe pour encapsuler ce dernier,
(d) récupérer les nanoparticules obtenues à la fin de l'étape (c).

La co-condensation entre le dérivé de porphyrine tel que défini ci-dessus, le composé de formule I et éventuellement le composé de formule II formant la matrice des nanoparticules de l'invention, peut être effectuée en présence d'un tensioactif pour obtenir la porosité du système.

A titre d'exemple, le tensioactif peut être le bromure de cétyltriméthylammonium, le chlorure de cétyltriméthylammonium, le bromure d'octadecyltrimethylammonium, le tosylate de cétyltriméthylammonium.

L'étape (a) du procédé de l'invention peut être mise en œuvre dans une solution aqueuse basique, notamment à pH comprise entre 8 et 13.

Les nanoparticules formées dans l'étape (a) peuvent être récupérées par toutes techniques connues de l'homme du métier, notamment par centrifugation.

La mise en œuvre de l'étape (c) permet d'encapsuler au moins un composé anticancéreux hydrophile et/ou hydrophobe dans les pores des nanoparticules récupérées à la fin de l'étape (b).

L'invention concerne également les nanoparticules d'organosilice mésoporeuses obtenues par le procédé de l'invention.

Les nanoparticules d'organosilice mésoporeuse de l'invention peuvent être utilisées en tant qu'agent photosensibilisant à des fins thérapeutiques ou de diagnostic. Ces nanoparticules peuvent être excitées par un photon ou deux photons. Selon l'objectif thérapeutique et/ou le type de maladie à traiter, il est possible d'exciter les nanoparticules de l'invention par un photon ou deux photons pour optimiser les résultats souhaités. Par exemple, pour traiter une surface relativement importante, il est avantageux d'utiliser l'excitation par un photon, alors que l'excitation des nanoparticules par deux photons permet de traiter les tissus très localisés.

Les nanoparticules de base peuvent être utilisés seules pour la thérapie photodynamique ou en tant qu'agent d'encapsulation pour la délivrance d'au moins un composé hydrophile et/ou hydrophobe, notamment d'un composé hydrophile.

Les nanoparticules de l'invention encapsulant au moins un composé anticancéreux hydrophile et/ou hydrophobe peuvent offrir une synergie d'activité anticancéreuse liée à la fois aux propriétés des composés anticancéreux et à la photosensibilité des nanoparticules de base.

Les nanoparticules d'organosilice mésoporeuses telles que décrites ci-dessus peuvent être utilisées à titre de médicament dans le traitement des cancers, des tumeurs, des maladies prolifératives cellulaires, ou des maladies de la peau.

Un médicament comprenant des nanoparticules de l'invention peut être activé par le rayonnement infra-rouge, qui pénètre mieux dans l'organisme que le rayonnement visible, et donc être utilisé non seulement pour traiter les cancers superficiels, mais également les tissus cancéreux situés plus profondément auxquels les agents photosensibilisants connus à ce jour ne peuvent pas accéder.

Parmi les différents cancers que les nanoparticules de l'invention sont susceptibles de traiter, on peut notamment citer le cancer du sein, du col de l'utérus, du colon, de l'épiderme, du poumon, de l'ovaire, de la prostate, le rétinoblastome ainsi que les mélanomes et l'ensemble des tumeurs solides qui inclut, mais n'est pas limité aux cancers du cou et de la tête, aux cancers digestifs, et toute tumeur bénigne ou cancéreuse.

Un objet de l'invention concerne des compositions pharmaceutiques comprenant les nanoparticules telles que décrites ci-dessus et un véhicule pharmaceutiquement acceptable.

L'homme du métier saura choisir un véhicule convenable selon ses connaissances générales selon les propriétés des nanoparticules de l'invention.

Les nanoparticules de l'invention peuvent être administrées localement ou par voie systémique. L'administration locale peut être effectuée notamment par injection de la composition de nanoparticules à proximité de la zone tumorale. Dans le cas des tumeurs superficielles, les compositions de nanoparticules peuvent être administrées par voie topique, sous une forme galénique appropriée (solution, suspension, pâte, patch). L'administration par voie générale peut être mise en œuvre par voie intraveineuse, intramusculaire, sous-cutanée, intrapéritonéale ou rectale. De telles formulations et leur mode de mise en œuvre sont bien connus de l'homme du métier. Le dosage de la composition en actif de nanoparticules et/ou celui de composé anticancéreux encapsulé est adapté en fonction du poids et de l'âge du patient, de la nature, de la localisation et du stade de développement de la tumeur, de la voie d'administration choisie et de la dose d'irradiation utilisée. La composition peut comprendre en outre tout autre actif connu pour le traitement des tumeurs et/ou de leurs symptômes. Elle comprend les composants classiques de la galénique adaptés au mode d'administration choisi. En particulier, elle peut être sous une forme galénique favorisant la vectorisation vers les tissus cibles. Pour le traitement des tissus internes, une fois après l'administration parentérale d'une composition pharmaceutique de l'invention, une source de lumière ou laser peut être introduite aux tissus à traiter à l'aide d'endoscopie ou des cathéters à fibres optiques.

L'invention a également pour objet un kit pour la détection d'une pathologie choisie parmi les cancers, les tumeurs et les maladies prolifératives cellulaires comprenant :
- des nanoparticules telles que décrites ci-dessus ou une composition de médicament les comprenant, et
- des moyens permettant une irradiation laser pulsé ou non du bleu au proche IR.

A titre d'exemple, ces moyens peuvent être notamment une diode laser ou un laser pulsé femtoseconde permettant d'émettre une lumière ayant une longueur d'onde de 400 nm à 1000 nm respectivement.

Du fait que les nanoparticules de l'invention sont capables d'émettre une lumière visible ou une fluorescence après irradiation mono-photonique ou bi-photonique, ces nanoparticules peuvent également être utilisées en tant qu'agent luminescent ou fluorescent dans le domaine de l'imagerie pour la détection ou le suivi par imagerie, notamment par microscopie biphotonique, des cancers, des tumeurs, des maladies prolifératives cellulaires, ou des maladies de la peau.

Notamment, les nanoparticules dans lesquelles sont intégrées les dérivés de porphyrine de formule A peuvent être excitées par deux photons et sont donc aptes à être utilisées en microscopie biphotonique.

On entend par "microscopie biphotonique" la microscopie confocale en fluorescence par excitation à deux photons, permettant de donner une image d'échantillon en haute résolution en 3 dimensions.

Les figures et les exemples ci-après ont pour objectif d'illustrer plus en détail la présente invention et ne signent nullement une limitation de la portée possible de la présente invention.
Figure 1: Graphique représentant le spectre UV-VIS d'une solution des nanoparticules CM238 obtenue après sa mise en suspension dans l'éthanol.
Figure 2: Imagerie en microscopie confocale biphotonique de cellules cancéreuses MCF-7 survivantes après 20h d'incubation avec les nanoparticules CM238. A gauche: fluorescence du marqueur membranaire ; au milieu: fluorescence émise par les cellules vivantes à 750 nm; à droite : superposition des deux images de fluorescence illustrées dans les figures à gauche et au milieu.
Figure 3: Pourcentage de cellules MCF-7 survivantes avant (barre noire) et après (barre blanche) l'excitation à 800 nm par 3 scans de 1,57 s à la puissance maximum à l'aide d'un microscope et un laser biphotonique, après 20 heures d'incubation dans un milieu de culture témoin ("Control") ou avec 80 µg/ml de nanoparticules CM238.
Figure 4 : Profile du relargage, dans l'eau, de la gemcitabine encapsulée, à partir des nanoparticules "CM238+gemcitabine". La flèche signifie l'addition d'HCI. L'axe des abscisses représente le temps en minute. L'axe des ordonnées représente la gemcitabine libérée.
Figure 5: Cytotoxicité des nanoparticules "CM238+gemcitabine" sur les cellules MCF-7 en l'absence d'excitation. L'axe des abscisses représente la concentration en nanoparticules exprimée en µg/mL. L'axe des ordonnées représente le pourcentage de cellules survivantes par le test au MTT.
Figure 6: Pourcentage de cellules MCF-7 survivantes avant (barre grise claire) et après (barre grise foncée) l'excitation à 800 nm par 3 scans à la puissance maximum à l'aide par microscopie biphotonique, après 20 heures d'incubation dans un milieu de culture témoin ("Control") ou avec 40 µg/ml de nanoparticules "CM238+gemcitabine".
Figure 7: Graphique représentant le spectre UV-VIS d'une solution des nanoparticules CM 240 obtenue après sa dispersion dans l'éthanol.
Figure 8: Pourcentage de cellules MCF-7 survivantes avant (barre grise claire) et après l'excitation (barre grise foncée) à 800 nm par 3 scans à la puissance maximum à l'aide de la microscopie biphotonique, après 20 heures d'incubation dans un milieu de culture témoin ("Control") ou avec 40 µg/ml ou 80 µg/ml de nanoparticules CM240.
Figure 9: Imagerie en microscopie confocale biphotonique de cellules cancéreuses MCF-7 survivantes après 20h d'incubation dans un milieu de culture contrôle (en haut) ou avec 80 µg/ml de nanoparticules CM240 (en bas). Colonne A : fluorescence du marqueur membranaire ; Colonne B :
   fluorescence émise par les nanoparticules CM240 dans les cellules vivantes à 800 nm; Colonne C : superposition des deux images de fluorescence illustrées dans les figures des colonnes A et B.
Figure 10: Cytotoxicité des nanoparticules CM240 sur les cellules MCF-7 en l'absence d'excitation. L'axe des abscisses représente la concentration en nanoparticules exprimée en µg/ml. L'axe des ordonnées représente le pourcentage de cellules survivantes par le test au MTT.
Figure 11 : Pourcentage de cellules MCF-7 survivantes avant (barre blanche) et après (barre noire) l'excitation par un laser monophotonique de puissance 10 mW à 405 nm pendant 10 min après 20 heures d'incubation dans un milieu de culture témoin ("Control") ou avec 80 µg/ml de nanoparticules CM238.
Figure 12 : Détection de ROS à partir de cellules MCF-7 incubées pendant 24h avec 80 µg/ml des nanoparticules CM 238.
Figure 13 : Détection de ROS à partir de cellules MCF-7 incubées pendant 24h avec 80 µg/ml des nanoparticules CM 240.
Figure 14 : Profile du relargage, dans l'eau, de la gemcitabine encapsulée, à partir des nanoparticules "CM240-b+gemcitabine". La flèche signifie l'addition d'HCI. L'axe des abscisses représente le temps en minute. L'axe des ordonnées représente la gemcitabine libérée.
Figure 15 : Graphique représentant le spectre UV-VIS d'une solution des nanoparticules PMOS1 obtenue après sa mise en suspension dans l'éthanol.
Figure 16 : Imagerie en microscopie confocale biphotonique de cellules cancéreuses MCF-7 survivantes après 20h d'incubation dans un milieu de culture contrôle (en haut) ou avec 80 µg/ml de nanoparticules PMOS1 (en bas). Colonne A : fluorescence des noyeaux ; Colonne B : fluorescence du marqueur membranaire ; Colonne C : fluorescence émise par les nanoparticules PMOS1 dans les cellules vivantes à 850 nm; Colonne D : superposition des deux images de fluorescence illustrées dans les figures des colonnes A, B et C.
Figure 17 : Pourcentage de cellules MCF-7 survivantes avant (barre blanche) et après (barre noire) l'excitation par un laser monophotonique de puissance 10 mW à 405 nm pendant 10 min après 20 heures d'incubation dans un milieu de culture témoin ("Control") ou avec 80 µg/ml de nanoparticules PMOS1.
Figure 18 : Profile du relargage, dans l'eau, de la gemcitabine encapsulée, à partir des nanoparticules "PMOS1+gemcitabine". La flèche signifie l'addition d'HCl. L'axe des abscisses représente le temps en minute. L'axe des ordonnées représente la gemcitabine libérée.

### Exemples

### 1. Matériels et méthodes

### 1.1. Préparation des nanoparticules CM238

250 mg de bromure de cetyltrimethylammonium et 875 µL de NaOH (2M) sont introduits dans 120 mL d'eau ultrapure. Le mélange est agité à 750 tour/min pendant 50 minutes à 80°C. La porphyrine de formule A1a (dans 1 mL d'éthanol absolu) est introduite simultanément en réaction avec le bis(triéthoxysilyl)éthane (10/90 en masse). La réaction est maintenue pendant 1h 45 minutes à 80°C. Puis les nanoparticules CM 238 obtenues sont centrifugées. Le tensioactif est extrait par une solution éthanolique de nitrate d'ammonium (6g/L). Les nanoparticules sont mises en suspension dans cette solution (50 mL) pendant 30 minutes aux ultrasons à 50°C et centrifugées à 20000 tour/min pendant 20 min. Le protocole est répété trois fois. Trois lavages avec éthanol sont ensuite réalisés. Les nanoparticules sont séchées sous vide.

### 1.2 Préparation des nanoparticules CM240

NaOH 2M (875 µl) et le bromure de cétyltriméthylammonium (250 mg) sont mélangés dans 120 mL d'eau à 80°C pendant 120 min. La porphyrine de formule A1a (1,40x10⁻² mmol, 23,8 mg diluée dans 1 mL d'EtOH), le bis(triéthoxysilyl)éthane (1,78 mmol) et le bis triéthoxysilylpropyl disulfure (1,3 mmol) (rapport 1/55/44 en moles) sont alors ajoutés. La réaction est maintenue pendant 2h à 80°C à 750 tour par minute. Puis les nanoparticules sont centrifugées à 20000 tours par minute pendant 15 min. Le tensioactif est extrait par une solution éthanoïque de nitrate d'ammonium (6g/L). Les nanoparticules sont mises en suspension dans cette solution (50 mL) pendant 30 minutes aux ultrasons à 50°C et centrifugées. Le protocole est répété trois fois. Les nanoparticules sont séchées sous vide.

### 1.3 Préparation des nanoparticules CM240-b

NaOH 2M (875 µl) et le bromure de cétyltriméthylammonium (250 mg) sont mélangés dans 120 mL d'eau à 80°C pendant 120 min. La porphyrine de formule A1a (1,40x10-2 mmol, 23,8 mg diluée dans 1 mL d'EtOH), le bis(triéthoxysilyl)éthane (1,78 mmol) et le bis triéthoxysilylpropyl disulfure (0,3 mmol) (rapport 1/83/16 en moles) sont alors ajoutés. La réaction est maintenue pendant 2h à 80°C à 750 tour par minute. Puis les nanoparticules sont centrifugées à 20000 tours par minute pendant 15 min. Le tensioactif est extrait par une solution ethanolique de nitrate d'ammonium (6g/L). Les nanoparticules sont mises en suspension dans cette solution (50 mL) pendant 30 minutes aux ultrasons à 50°C et centrifugées. Le protocole est répété trois fois. Les nanoparticules sont séchées sous vide.

### 1.4. Préparation des nanoparticules PMOS1

NaOH 2M (437 µl) et le bromure de cétyltriméthylammonium (125 mg) sont mélangés dans 60 mL d'eau à 80°C pendant 120 min. La porphyrine de formule B (1,3 x10-2 mmol, 12 mg diluée dans 1 mL d'EtOH) et le bis(triéthoxysilyl)éthane (0,89 mmol) sont alors ajoutés. La réaction est maintenue pendant 2h à 80°C à 750 tour par minute. Puis les nanoparticules sont centrifugées à 20000 tours par minute pendant 15 min. Le tensioactif est extrait par une solution éthanoïque de nitrate d'ammonium (6g/L). Les nanoparticules sont mises en suspension dans cette solution (50 mL) pendant 30 minutes aux ultrasons à 50°C et centrifugées. Le protocole est répété trois fois. Les nanoparticules sont séchées sous vide.

### 1.5. Encapsulation de la gemcitabine

1,6 mg de nanoparticules CM238 sont mises en suspension avec 1,9 mg de gemcitabine dans 2 mL d'eau (pH=7.4) pendant 24 h. Puis les nanoparticules sont centrifugées et lavées 4 fois à l'eau et séchées sous vide. Les surnageants sont collectés afin de déterminer la quantité de médicament encapsulée dans les nanoparticules.

### 1.6. Spectre UV-Vis des nanoparticules

1 mg de nanoparticules sont dispersées dans 1 mL d'EtOH. Le spectre UV-Vis des nanoparticules est observé à l'aide d'un spectromètre UV-Vis.

### 1.7. Imagerie en microscopie confocale biphotonique des cellules

Les cellules MCF-7 du cancer du sein sont incubées pendant 20h avec les nanoparticules. 15 minutes avant l'imagerie, la membrane des cellules est teintée avec un colorant. Les nanoparticules sont observées à 750 nm avec un microscope confocal biphotonique et une faible puissance laser (5% de la puissance totale (3W) délivrée par le laser pulsé femtoseconde Chameleon).

### 1.8. Cinétique de relargage de la gemcitabine encapsulée

Les nanoparticules "NPs+gemcitabine" sont introduite au fond d'une cuve UV puis on ajoute une solution aqueuse à pH 7,4 sans agitation. La concentration de gemcitabine libérée dans la solution est mesurée après 10, 20, 30 minutes. HCl est ajouté dans la solution après 50 minutes. La concentration de la gemcitabine libérée dans la solution est ensuite mesurée à 60, 90, 120, 130 et 140 minutes.

### 1.9. Cytotoxicité des nanoparticules

Les cellules cancéreuses MCF-7 sont incubées pendant 20h avec des nanoparticules de différentes concentrations. La cytotoxicité des nanoparticules est mesurée sans ou après l'excitation. L'excitation est faite par microscope confocal LSM 780 Zeiss (objectifx10) à 800 nm.

La quantification de cellules vivantes est obtenue par le test MTT (test de survie cellulaire) après 48 heures d'irradiation. Le test MTT est effectué selon un protocole conventionnel (Mosmann, Journal of Immunological Methods, 1983, 65 (1-2): 55-63).

### 1.10 Détermination de la production de ROS

La génération de ROS est examinée dans les cellules en utilisant le kit DCFDA (diacétate de dichlorodihydrofluorescéine). En contact avec des espèces ROS, le DCFDA non fluorescent est oxydé en dihydrofluorescéine fluorescente (DCF).

Avant l'irradiation biphotonique, le DCFDA a donc été incubé pendant 45 min avec les cellules après endocytose des nanoparticules. L'expérience ne révèle aucune fluorescence significative sans irradiation tandis qu'un signal fluorescent élevé est détecté lors de l'irradiation des cellules, ce qui a confirmé la production de ROS sur TPE. L'intensité de la fluorescence est proportionnelle à la quantité de ROS générée (détection à 535 nm).

### 2. Résultats

### 2.1. Analyse des nanoparticules CM238

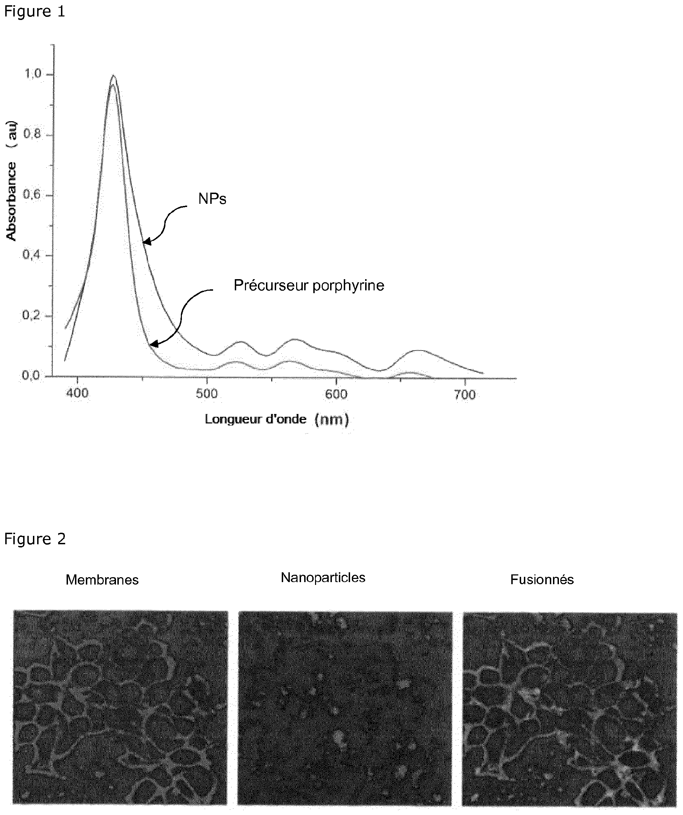

Les nanoparticules CM 238 obtenues ont une surface spécifique très élevée (832 m² g⁻¹) et une taille de pore de 3 nm. Elles sont monodisperses avec un diamètre de 200-250 nm. Elles se dispersent dans l'eau ou l'éthanol. Les porphyrines dans ces nanoparticules s'agrègent en agrégats J avec un décalage du spectre UV-Vis vers le rouge (Figure 1).

Ces particules peuvent être utilisées pour l'imagerie biphotonique de cellules cancéreuses. Les cellules MCF-7 du cancer du sein sont incubées pendant 20h avec les nanoparticules CM238 et ensuite observées à 750 nm avec un microscope confocal et une faible puissance laser (5% de la puissance totale (3W) délivrée par le laser pulsé femtoseconde Chaméléon). Cette expérience montre que les nanoparticules sont internalisées dans les cellules MCF-7 (Figure 2).

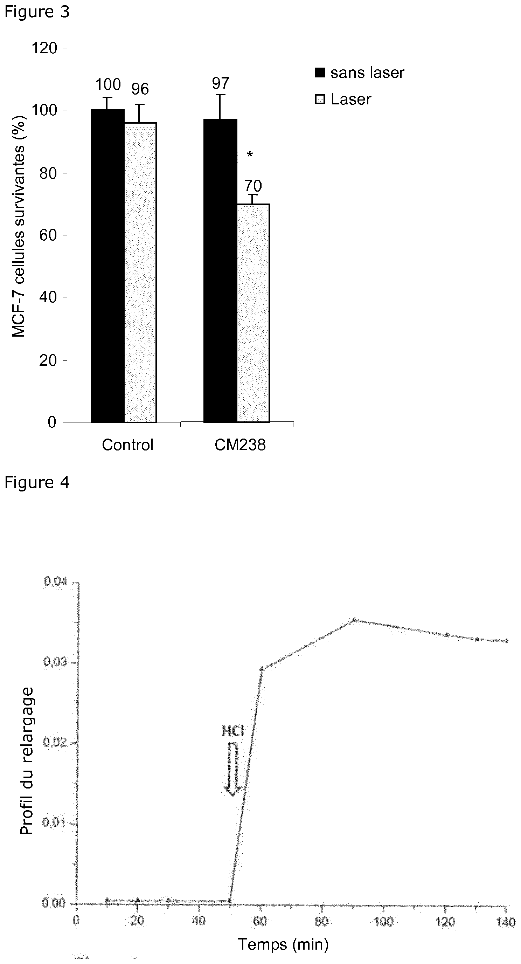

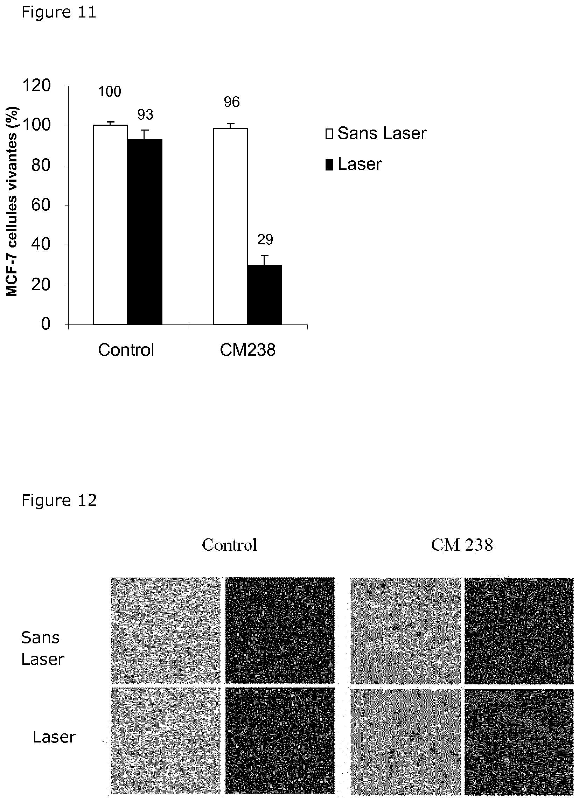

Après 20h d'incubation des cellules cancéreuses MCF-7 avec 80µg/ml de nanoparticules CM238, les cellules sont irradiées par 3 scans de 1,57s à 800 nm par microscopie confocale biphotonique. Environ 27% des cellules cancéreuses sont détruites après irradiation (Figure 3). Dans les mêmes conditions d'incubation, les cellules sont irradiées pendant 10 min par un laser monophotonique à 405 nm et une puissance de 10 mW. 71% des cellules sont détruites (Figure 11).

La production de ROS concorde avec l'irradiation biphotonique et est proportionnelle à l'intensité de la fluorescence (figure 12).

### 2.2. Analyse des nanoparticules "CM238+gemcitabine"

Les nanoparticules de base CM238 dont la matrice est formée par un dérivé de porphyrine de formule A1a et le bis(triethoxysilyl)éthane sont obtenues selon la méthode décrite dans la partie 1.1.

La gemcitabine est encapsulée dans les nanoparticules CM238 selon la méthode décrite dans la partie 1.2.

La charge en gemcitabine en masse par rapport au poids des nanoparticules de base est de 50%. Cela signifie que la gemcitabine est encapsulée efficacement dans les nanoparticules de base.

La délivrance de la gemcitabine est sensible au pH. A pH 7,4, les nanoparticules en suspension ne relarguent pas la gemcitabine, alors qu'à pH 5,5 (pH des cellules cancéreuses), il y a une délivrance nette de la gemcitabine (Figure 4).

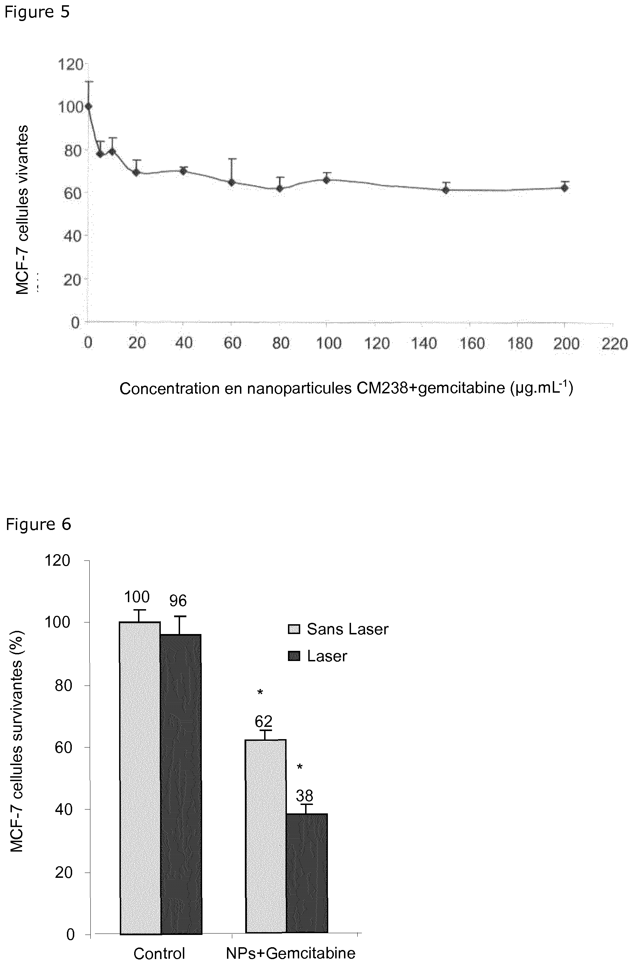

La cytotoxicité des nanoparticules "CM238+gemcitabine" à différentes concentrations sans excitation a été testée sur des cultures cellulaires MCF-7. Après trois jours d'incubation, jusqu'à 40% des cellules sont détruites (Figure 5). Ces résultats montrent que la gemciabine est délivrée efficacement dans les cellules cancéreuses.

Lorsque les nanoparticules "CM238+gemcitabine" sont incubées à une concentration de 40 µg.mL⁻¹ pendant 20 heures avec les cellules MCF-7 du cancer du sein, après l'irradiation à 800 nm avec 3 scans à la puissance maximum, 62% des cellules sont détruites du fait du double traitement par la gemcitabine et la thérapie photodynamique (figure 6).

### 2.3. Analyse des nanoparticules CM240

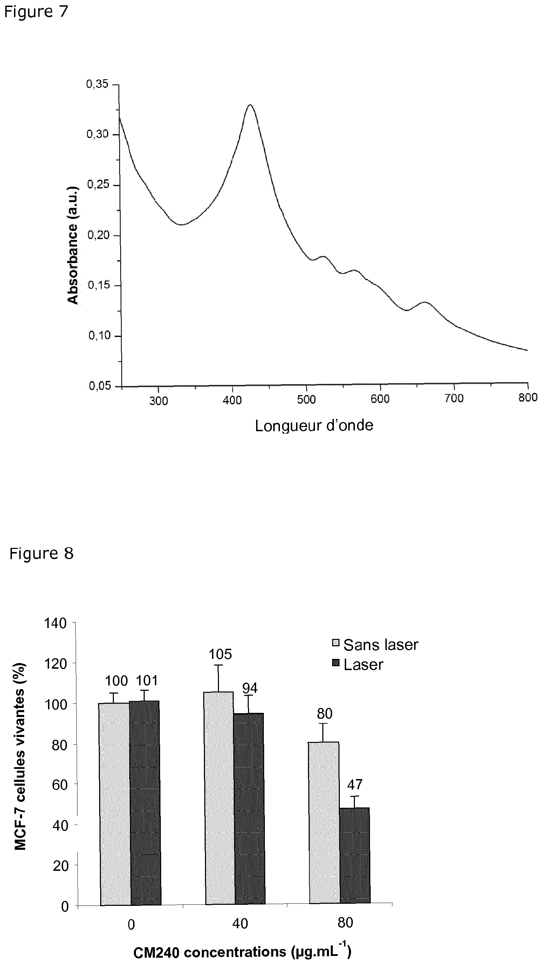

Les nanoparticules CM 240 obtenues ont une surface spécifique très élevée (950 m² g⁻¹) et une taille de pore de 2,2 nm. Elles sont monodisperses. Elles se dispersent dans l'eau ou l'éthanol. Les porphyrines dans ces nanoparticules s'agrègent en agrégats J avec un décalage du spectre UV-Vis vers le rouge (Figure 7).

A 80 µg/ml, après 20h d'incubation avec des cellules cancéreuses MCF-7, les nanoparticules CM240 après excitation à 800 nm par microscopie confocale biphotonique LSM 780 Zeiss (objectifx10) peuvent détruire 53% des cellules cancéreuses (Figure 8). En revanche, à 40 µg/ml, les nanoparticules CM240 ne montrent pas une cytotoxicité significative.

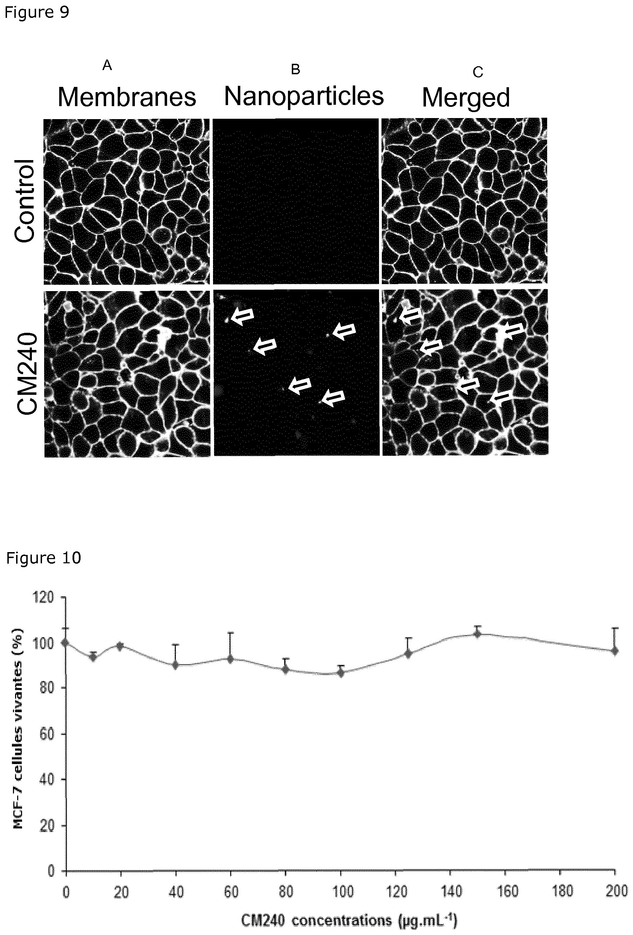

Les cellules survivantes après 20h d'incubation avec 80µg/ml de nanoparticules CM240 sont observées par microscope confocale biphotoniqueLSM 780 Zeiss (objectifx63). On observe que les nanoparticules sont entrées dans les cellules (Figure 9).

Par ailleurs, lorsque des cellules MCF-7 sont traitées par des concentrations croissantes de CM240, en l'absence d'excitation, les nanoparticules CM240 ne sont pas toxiques pour les cellules (Figure 10).

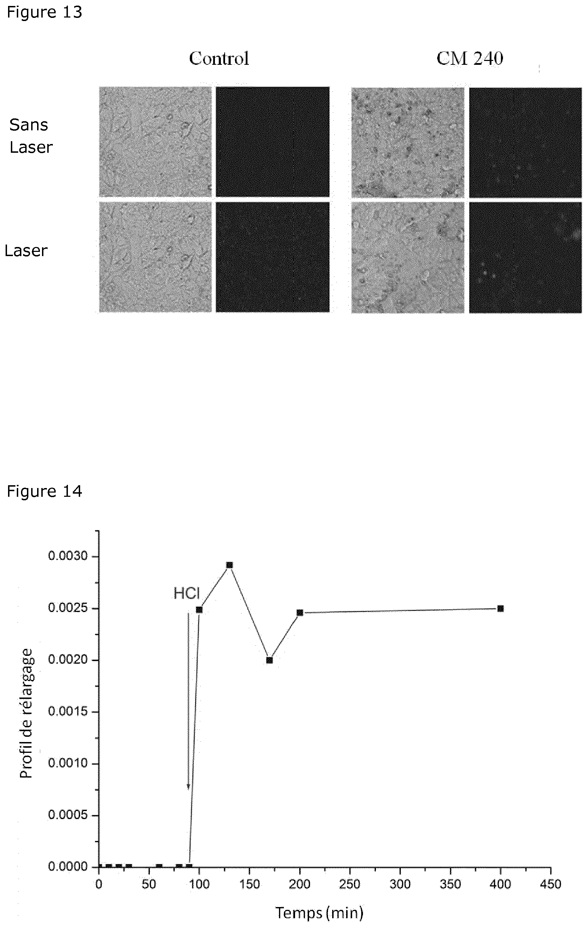

La production de ROS concorde avec l'irradiation biphotonique et est proportionnelle à l'intensité de la fluorescence (figure 13).

### 2.4. Analyse des nanoparticules "CM240-b+gemcitabine"

Les nanoparticules de base CM240-b dont la matrice est formée par un dérivé de porphyrine de formule A1a et le bis(triethoxysilyl)éthane ainsi que bis triéthoxysilylpropyl disulfure sont obtenues selon la méthode décrite dans la partie 1.3.

La gemcitabine est encapsulée dans les nanoparticules CM240-b selon la méthode décrite dans la partie 1.5. La charge en gemcitabine en masse par rapport au poids des nanoparticules de base est de 98%. Cela signifie que la gemcitabine est encapsulée efficacement dans les nanoparticules de base.

La délivrance de la gemcitabine est sensible au pH. A pH 7,4, les nanoparticules en suspension ne relarguent pas la gemcitabine, ainsi qu'à pH 5,5 (pH des cellules cancéreuses) (Figure 14).

### 2.5. Analyse des nanoparticules PMOS1

Les nanoparticules PMOS1 obtenues ont une surface spécifique très élevée (892 m2 g-1) et une taille de pore de 3 nm. Elles sont monodisperses avec un diamètre moyen de 305 nm. Elles se dispersent dans l'eau ou l'éthanol.

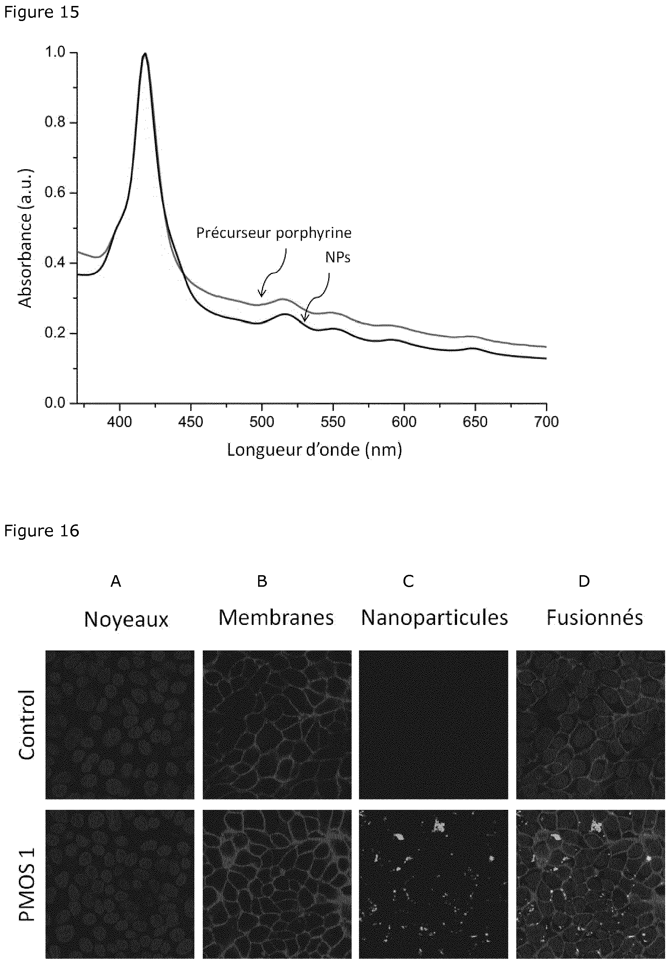

Les porphyrines dans ces nanoparticules ne présentent pas de décalage dans spectre UV-Vis en comparaison avec leur dérivé porphyrine de formule B (Figure 15). Ces particules peuvent être utilisées pour l'imagerie monophotonique de cellules cancéreuses. Les cellules MCF-7 du cancer du sein sont incubées pendant 20h avec les nanoparticules PMOS1 et ensuite observées à 850 nm.

Cette expérience montre que les nanoparticules sont internalisées dans les cellules MCF-7 (Figure 16).

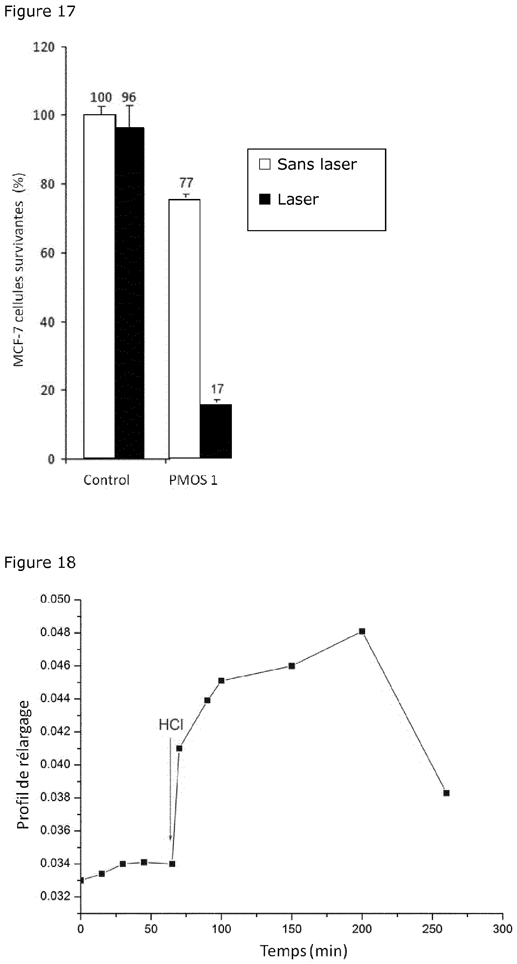

Après 20h d'incubation des cellules cancéreuses MCF-7 avec 80µg/ml de nanoparticules PMOS1, les cellules sont irradiées à 405 nm par un laser monophotonique et une puissance de 10 mW. Environ 80% des cellules cancéreuses sont détruites après irradiation (Figure 17).

### 2.6. Analyse des nanoparticules "PMOs1+gemcitabine"

Les nanoparticules de base PMOS1 dont la matrice est formée par un dérivé de porphyrine de formule B et le bis(triethoxysilyl)éthane sont obtenues selon la méthode décrite dans la partie 1.4.

La gemcitabine est encapsulée dans les nanoparticules PMOS1 selon la méthode décrite dans la partie 1.5. La charge en gemcitabine en masse par rapport au poids des nanoparticules de base est de 72%. Cela signifie que la gemcitabine est encapsulée efficacement dans les nanoparticules de base.

La délivrance de la gemcitabine est sensible au pH. A pH 7,4, les nanoparticules en suspension ne relarguent pas la gemcitabine, ainsi qu'à pH 5,5 (pH des cellules cancéreuses) où seulement 0,5% est relargué (Figure 18).

## Revendications

1. Nanoparticules d'organosilice mésoporeuses formées par les éléments comprenant ou constitués par :
(i) un dérivé de porphyrine choisi parmi :
- un composé de formule A 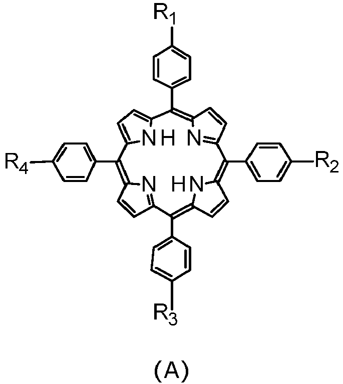 dans laquelle :
- Soit R₁, R₂, R₃, et R₄ correspondent tous à 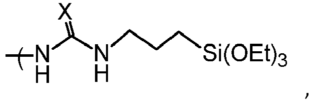 où X est l'atome d'oxygène ou l'atome de soufre,
- Soit R1, R2, R3, et R4 correspondent tous à 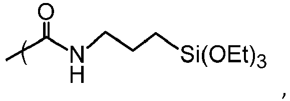
- un composé de formule B, C ou D, dans lesquelles Z est choisi parmi l'atome d'oxygène ou l'atome de soufre 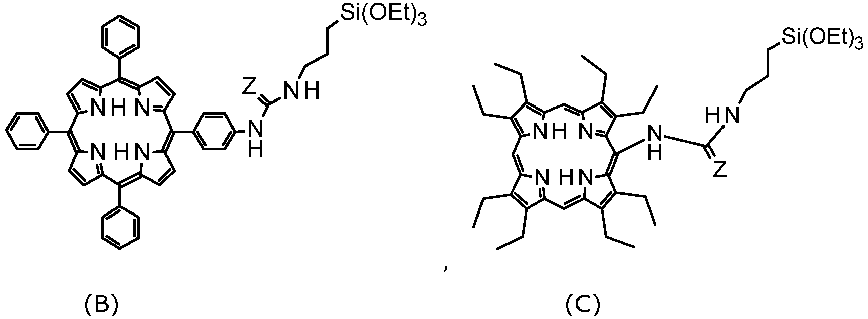 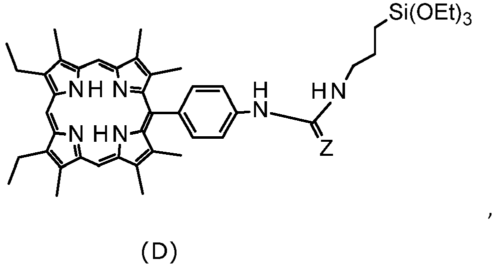 ou
- un composé E, F, G 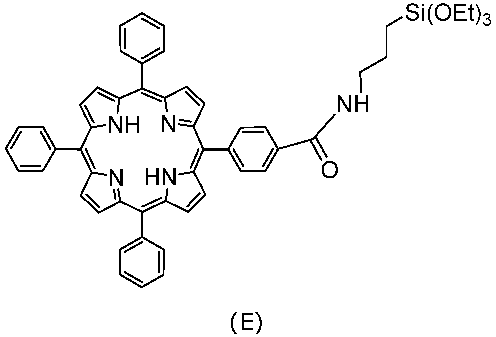 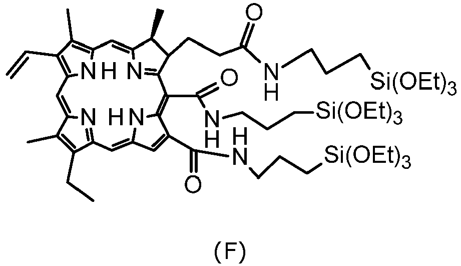 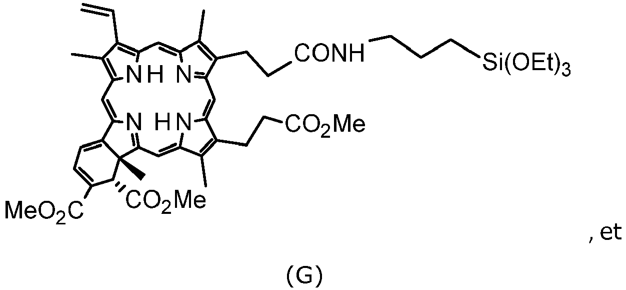
(ii) un composé de formule I :
(EtO)₃Si(CH₂)ₙSi(OEt)₃,
dans laquelle n représente un entier choisi entre 1 et 10,
et éventuellement
(iii) un composé de formule II
(EtO)₃Si(CH₂)₃-(S-S)ₘ-(CH₂)₃-Si(OEt)₃
dans laquelle m est un nombre entier égal à 2 ou 4,
lesdites nanoparticules éventuellement encapsulant au moins un composé anticancéreux hydrophile et/ou hydrophobe.

2. Nanoparticules selon la revendication 1, ledit composé anticancéreux hydrophile est choisi parmi la gemcitabine, la gemcitabine monophosphate, le 5-fluorouracile, la cytarabine, le topotécane, l'irinotécane, ou l'oxalylplatine ; ledit composé anticancéreux hydrophobe est choisi parmi choisi parmi la doxorubicine, le paclitaxel, ou la camptothécine.

3. Nanoparticules selon la revendication 1 ou 2, dans lesquelles le dérivé de porphyrine est un composé de formule A et s'agrège en agrégat.

4. Nanoparticules selon la revendication 3, formées par les éléments constitués par
(i)un dérivé de porphyrine tel que défini dans la revendication 1, notamment un dérivé de porphyrine de formule ci-après 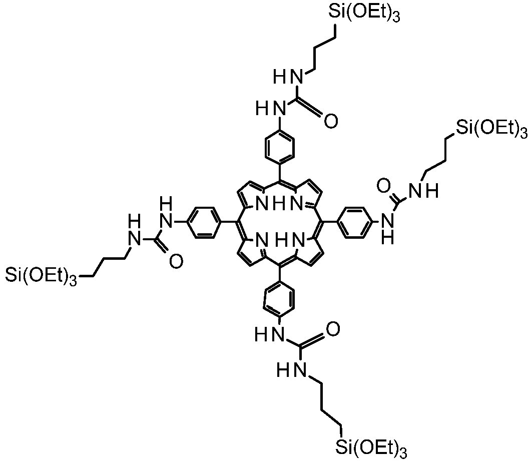 ou un dérivé de porphyrine de formule B, et
(ii) un composé de formule I tel que défini selon la revendication 1.

5. Nanoparticules selon la revendication 3, formées par les éléments constitués par
(i) un dérivé de porphyrine tel que défini dans la revendication 1, notamment un dérivé de porphyrine de formule ci-après 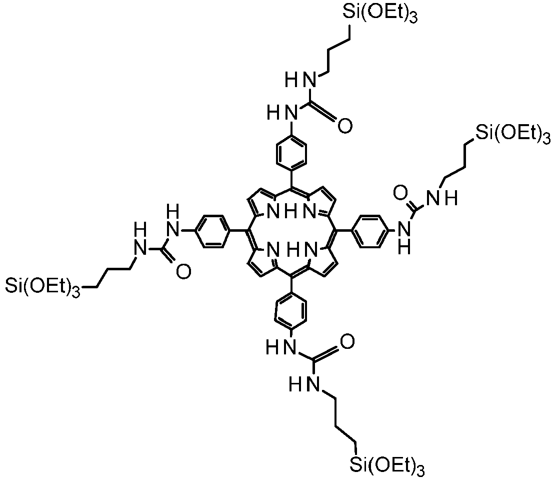 ou un dérivé de porphyrine de formule B, et
(ii) un composé de formule I tel que défini selon la revendication 1, lesdites nanoparticules encapsulant la gemcitabine ou la gemcitabine monophosphate.

6. Nanoparticules selon la revendication 3, formées par les éléments constitués par
(i) un dérivé de porphyrine tel que défini dans la revendication 1, notamment un dérivé de porphyrine de formule ci-après 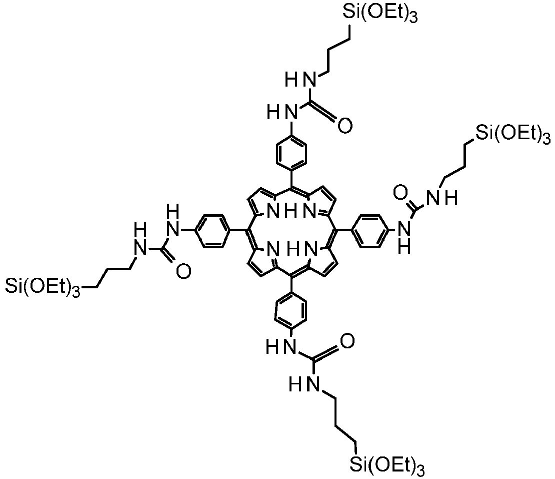 ou un dérivé de porphyrine de formule B,
(ii) un composé de formule I tel que défini selon la revendication 1,
(iii) un composé de formule II tel que défini selon la revendication 1.

7. Nanoparticules selon la revendication 3, formées par les éléments constitués par
(i) un dérivé de porphyrine tel que défini dans la revendication 1, notamment un dérivé de porphyrine de formule 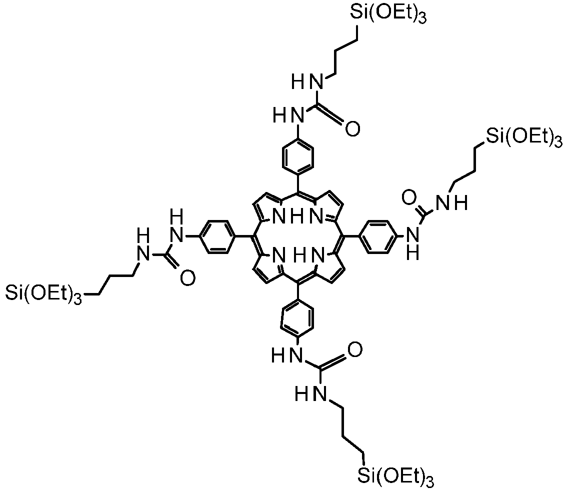 ou un dérivé de porphyrine de formule B,
(ii) un composé de formule I tel que défini selon la revendication 1,
(iii) un composé de formule II tel que défini selon la revendication 1, lesdites nanoparticules encapsulant la gemcitabine ou la gemcitabine monophosphate.

8. Nanoparticules selon l'une quelconque des revendications 1 à 7, dans lesquelles le rapport en moles entre le dérivé de porphyrine et le composé de formule I est entre 2 :98 à 20 :80.

9. Nanoparticules selon l'une quelconque des revendications 1 à 3, 5 et 7, dans lesquelles la charge en composé anticancéreux hydrophile ou hydrophobe tel que défini selon la revendication 1 est de 2% à 100%, notamment de 40%, en masse par rapport à la masse initiale des nanoparticules avant l'encapsulation dudit composé anticancéreux.

10. Nanoparticules selon l'une quelconque des revendications 1 à 9, dont le diamètre de particules est de 20 à 400 nm, avantageusement de 50 à 300 nm, plus avantageusement de 200-250nm ; la superficie spécifique est de 100-1500 m²/g, avantageusement de 800-1000 m²/g.

11. Nanoparticules selon l'une quelconque des revendications 1 à 10, à titre de médicament dans le traitement des cancers, des tumeurs, les maladies prolifératives cellulaires, ou les maladies de la peau.

12. Nanoparticules selon l'une quelconque des revendications 1 à 10, pour leur utilisation en tant qu'agent luminescent ou fluorescent après irradiation dans la détection ou le suivi par imagerie, notamment par microscopie biphotonique, des cancers, des tumeurs, les maladies prolifératives cellulaires, ou les maladies de la peau.

13. Nanoparticules selon l'une quelconque des revendications 1 à 10 pour leur utilisation en tant qu'agent photosensibilisant.

14. Composition pharmaceutique comprenant des nanoparticules selon l'une quelconque des revendications 1 à 10 et un véhicule pharmaceutiquement acceptable.

15. Procédé de fabrication de nanoparticules selon l'une quelconque des revendications 1 à 10, ce procédé comprenant les étapes consistant à:
(a) faire réagir en solution aqueuse basique à une température de 50°C à 90°C en présence d'un tensioactif, les composés comprenant :
(i) un dérivé de porphyrine choisi parmi :
- un composé de formule A 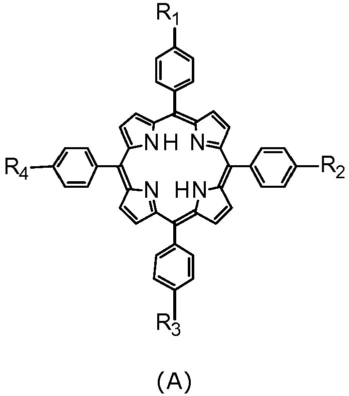 dans laquelle :
- Soit R₁, R₂, R₃, et R₄ correspondent tous à 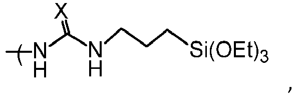 où X est l'atome d'oxygène ou l'atome de soufre,
- Soit R₁, R₂, R₃, et R₄ correspondent tous à 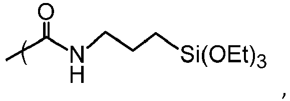
- un composé de formule B, C ou D, dans lesquelles Z est choisi parmi l'atome d'oxygène ou l'atome de soufre 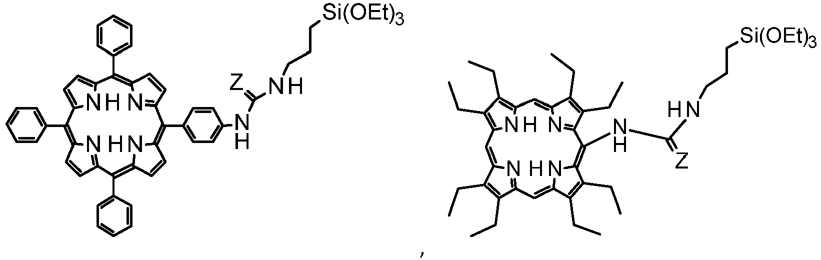 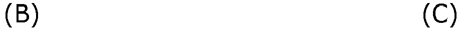 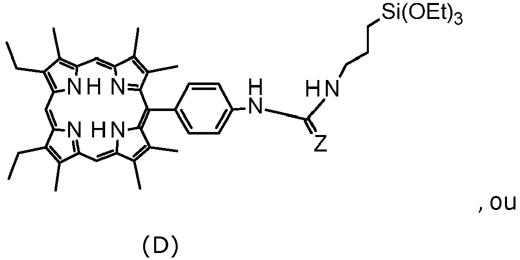
- un composé E, F, G 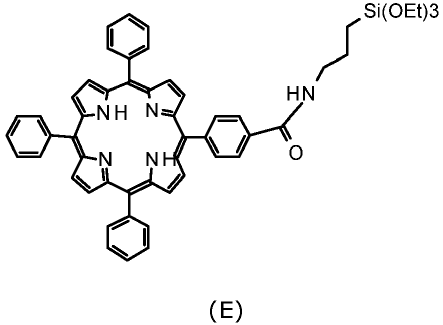 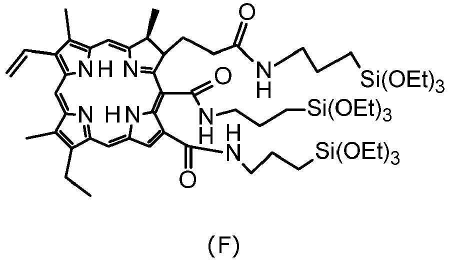 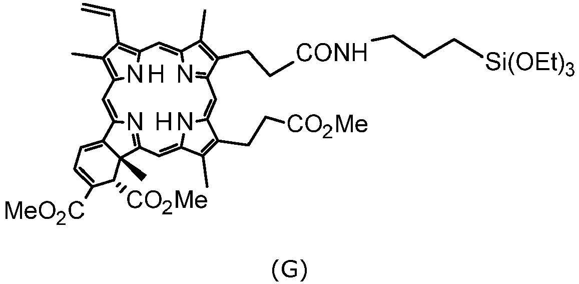
(ii) un composé de formule I ci-après
(EtO)₃Si(CH₂)ₙSi(OEt)₃,
dans laquelle n représente un entier choisi entre 1 et 10, et éventuellement
(iii) un composé de formule II
(EtO)₃Si(CH₂)₃-(S-S)ₘ-(CH₂)₃-Si(OEt)₃
dans laquelle m est un nombre entier égal à 2 ou 4, et
(b) récupérer les nanoparticules formées dans l'étape précédente, et éventuellement,
(c) faire réagir dans un solvant les nanoparticules obtenues dans l'étape (b) avec au moins un composé anticancéreux hydrophile et/ou hydrophobe pour encapsuler le dernier,
(d) récupérer les nanoparticules obtenues à la fin de l'étape (c).

16. Nanoparticules mésoporeuses obtenues par un procédé selon la revendication 15.

17. Kit pour la détection, d'une pathologie choisie parmi les cancers, les tumeurs, les maladies prolifératives cellulaires comprenant :
- des nanoparticules selon la revendication 1 à 10 ou une composition de médicament les comprenant, et
- des moyens permettant une irradiation LED, laser du bleu au proche IR.

## Patentansprüche

1. Mesoporöse Organosilica-Nanopartikel, gebildet von den Elementen, die enthalten oder bestehen aus:
(i) ein/einem Porphyrinderivat, ausgewählt aus:
- einer Verbindung der Formel A 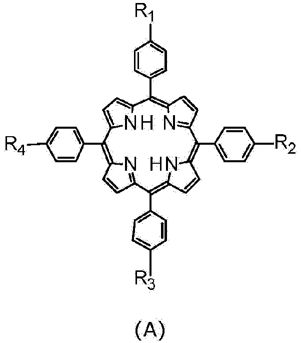 in der:
- R₁, R₂, R₃ und R₄ alle jeweils entsprechen 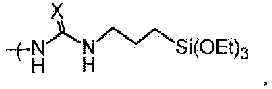 wobei X das Sauerstoffatom oder das Schwefelatom ist,
- R₁, R₂, R₃ und R₄ alle jeweils entsprechen 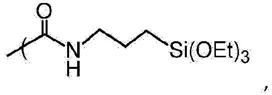
- einer Verbindung der Formel B, C oder D, bei denen Z ausgewählt ist aus dem Sauerstoffatom oder dem Schwefelatom 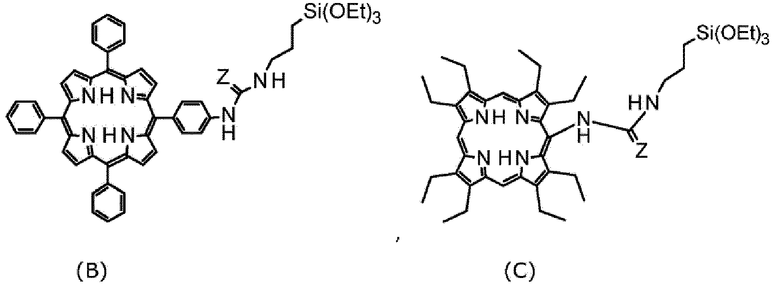 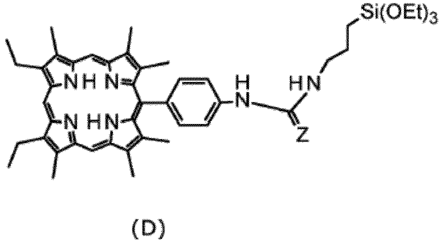 , oder
- einer Verbindung E, F, G 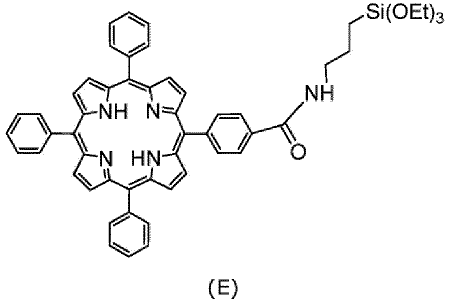 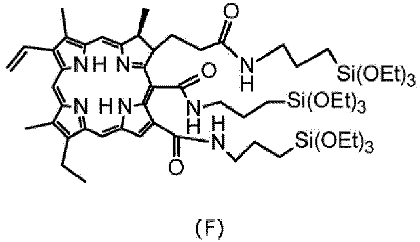 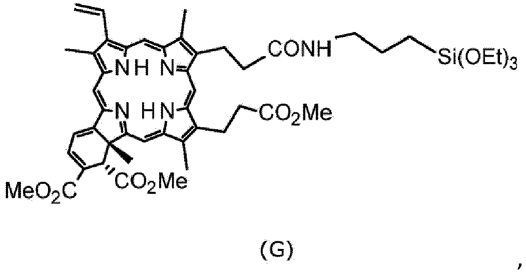 und
(ii) eine/einer Verbindung der Formel I:
(EtO)₃Si(CH₂)ₙSi(OEt)₃,
in der n eine ganze Zahl, ausgewählt zwischen 1 und 10, ist,
und gegebenenfalls
(iii) eine/einer Verbindung der Formel II:
(EtO)₃Si(CH₂)₃-(S-S)ₘ-(CH₂)₃-Si(OEt)₃,
in der m eine ganze Zahl gleich 2 oder 4 ist,
wobei die Nanopartikel gegebenenfalls mindestens eine hydrophile und/oder hydrophobe Antikrebs-Verbindung einkapseln.

2. Nanopartikel nach Anspruch 1, wobei die hydrophile Antikrebs-Verbindung ausgewählt ist aus Gemcitabin, Gemcitabinmonophosphat, 5-Fluoruracil, Cytarabin, Topotecan, Irinotecan oder Oxalylplatin; die hydrophobe Antikrebs-Verbindung ausgewählt ist aus Doxorubicin, Paclitaxel oder Camptothecin.

3. Nanopartikel nach Anspruch 1 oder 2, wobei das Porphyrinderivat eine Verbindung der Formel A ist und sich zu einem Aggregat häuft.

4. Nanopartikel nach Anspruch 3, gebildet durch die Elemente, die bestehen aus
(i) einem Porphyrinderivat, wie es im Anspruch 1 definiert ist, insbesondere einem Porphyrinderivat der folgenden Formel 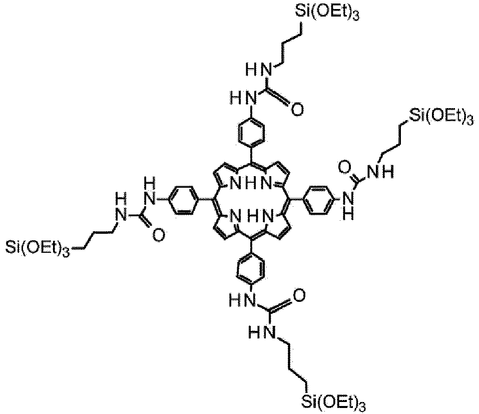 oder einem Porphyrinderivat der Formel B und
(ii) einer Verbindung der Formel I, wie nach Anspruch 1 definiert.

5. Nanopartikel nach Anspruch 3, gebildet durch die Elemente, die bestehen aus
(i) einem Porphyrinderivat, wie es im Anspruch 1 definiert ist, insbesondere einem Porphyrinderivat der folgenden Formel 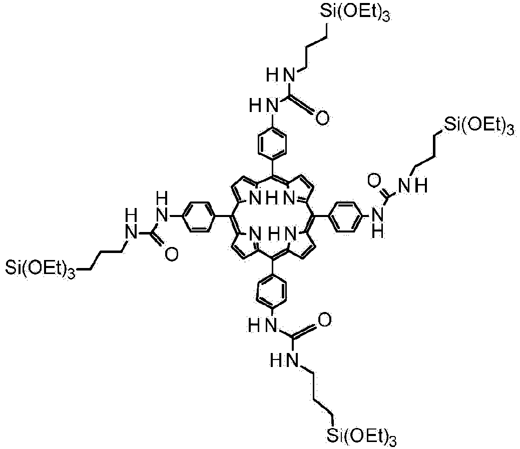 oder einem Porphyrinderivat der Formel B und
(ii) einer Verbindung der Formel I, wie nach Anspruch 1 definiert, wobei die Nanopartikel das Gemcitabin oder das Gemcitabinmonophosphat einkapseln.

6. Nanopartikel nach Anspruch 3, gebildet durch die Elemente, die bestehen aus
(i) einem Porphyrinderivat, wie es im Anspruch 1 definiert ist, insbesondere einem Porphyrinderivat der folgenden Formel 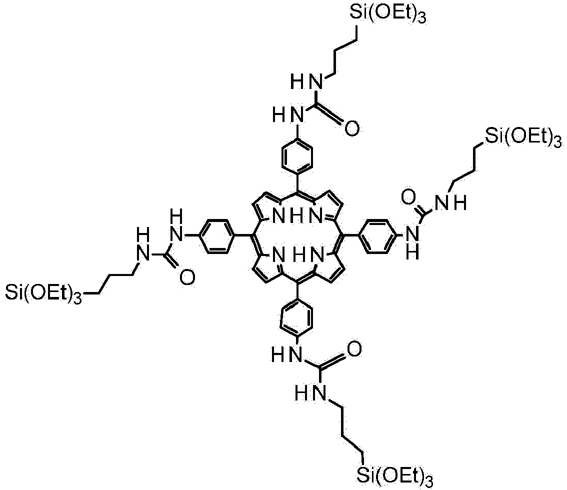 oder einem Porphyrinderivat der Formel B und
(ii) einer Verbindung der Formel I, wie nach Anspruch 1 definiert,
(iii) einer Verbindung der Formel II, wie nach Anspruch 1 definiert.

7. Nanopartikel nach Anspruch 3, gebildet durch die Elemente, die bestehen aus
(i) einem Porphyrinderivat, wie es im Anspruch 1 definiert ist, insbesondere einem Porphyrinderivat der folgenden Formel 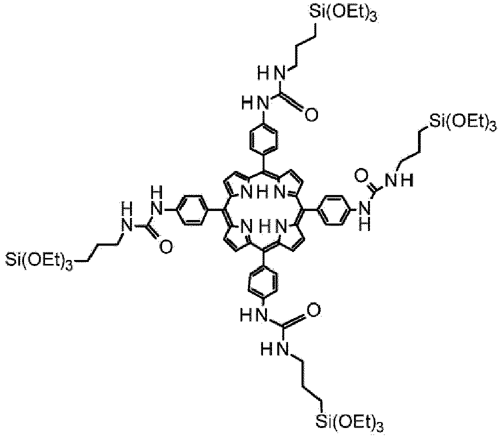 oder einem Porphyrinderivat der Formel B und
(ii) einer Verbindung der Formel I, wie nach Anspruch 1 definiert,
(iii) einer Verbindung der Formel II, wie nach Anspruch 1 definiert, wobei die Nanopartikel das Gemcitabin oder das Gemcitabinmonophosphat einkapseln.

8. Nanopartikel nach einem beliebigen der Ansprüche 1 bis 7, bei denen das Molverhältnis zwischen dem Porphyrinderivat und der Verbindung der Formel I zwischen 2:98 bis 20:80 liegt.

9. Nanopartikel nach einem beliebigen der Ansprüche 1 bis 3, 5 und 7, bei denen der Anteil an hydrophiler oder hydrophober Antikrebs-Verbindung, wie nach Anspruch 1 definiert, 2% bis 100%, insbesondere 40%, hinsichtlich der Masse in Bezug auf die Anfangsmasse der Nanopartikel vor der Verkapselung der Antikrebs-Verbindung ist.

10. Nanopartikel nach einem beliebigen der Ansprüche 1 bis 9, deren Partikeldurchmesser von 20 bis 400 nm, vorzugsweise von 50 bis 300 nm, noch bevorzugter von 200 bis 250 ist; wobei die spezifische Oberfläche von 100 bis 1500 m²/g, vorzugsweise von 800 bis 1000 m²/g, ist.

11. Nanopartikel nach einem beliebigen der Ansprüche 1 bis 10 als Medikament bei der Behandlung von Krebs, Tumoren, proliferativen zellulären Krankheiten oder Hautkrankheiten.

12. Nanopartikel nach einem beliebigen der Ansprüche 1 bis 10 für ihre Verwendung als lumineszierendes oder fluoreszierendes Agens nach Bestrahlung in der Detektion oder der Bildgebungsnachsorge, insbesondere durch biphotonische Mikroskopie, Mikroskopie des Krebses, der Tumore, der proliferativen zellulären Krankheiten oder Hautkrankheiten.

13. Nanopartikel nach einem der Ansprüche 1 bis 10 für ihre Verwendung als Photosensibilisator.

14. Pharmazeutische Zusammensetzung, Nanopartikel nach einem beliebigen der Ansprüche 1 bis 10 und einen pharmazeutisch verträglichen Träger umfassend.

15. Verfahren zur Herstellung von Nanopartikeln nach einem beliebigen der Ansprüche 1 bis 10, wobei dieses Verfahren die Schritte umfasst, die darin bestehen:
(a) in einer basischen wässrigen Lösung bei einer Temperatur von 50°C bis 90°C in Anwesenheit eines Tensids die Verbindungen reagieren zu lassen, die enthalten:
(i) ein Porphyrinderivat, ausgewählt aus:
- einer Verbindung der Formel A 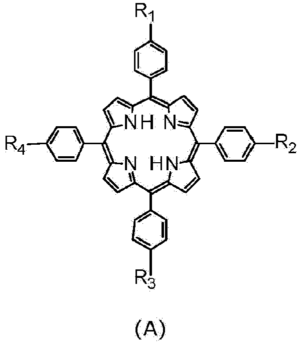 in der:
- R₁, R₂, R₃ und R₄ alle jeweils entsprechen 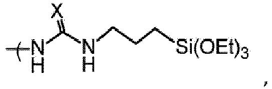 wobei X das Sauerstoffatom oder das Schwefelatom ist,
- R₁, R₂, R₃ und R₄ alle jeweils entsprechen 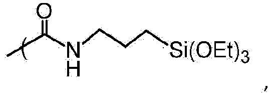
- einer Verbindung der Formel B, C oder D, bei denen Z ausgewählt ist aus dem Sauerstoffatom oder dem Schwefelatom 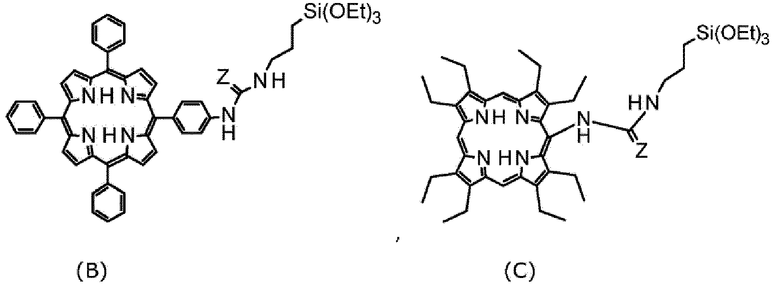 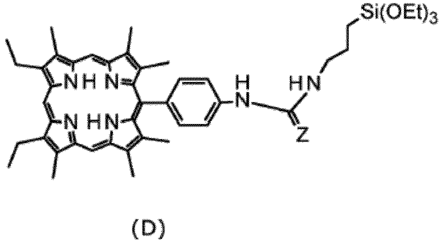 , oder
- eine Verbindung E, F, G 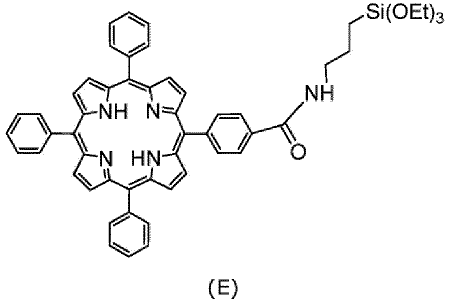 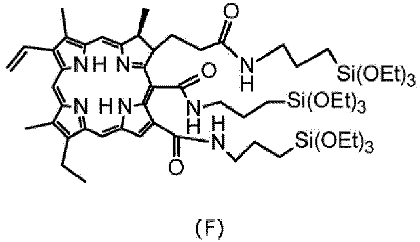 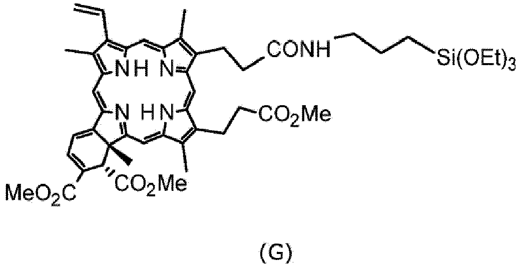
(ii) eine Verbindung der folgenden Formel I:
(EtO)₃Si(CH₂)ₙSi(OEt)₃,
in der n eine ganze Zahl, ausgewählt zwischen 1 und 10, ist,
und gegebenenfalls
(iii) eine Verbindung der Formel II:
(EtO)₃Si(CH₂)₃-(S-S)ₘ-(CH₂)₃-Si(OEt)₃,
in der m eine ganze Zahl gleich 2 oder 4 ist,
(b) Rückgewinnen der in dem vorherigen Schritt gebildeten Nanopartikel und gegebenenfalls
(c) in einem Lösungsmittel Reagieren lassen der in dem Schritt (b) erhaltenen Nanopartikel mit mindestens einer hydrophilen und/oder hydrophoben Antikrebs-Verbindung, um letztere einzukapseln,
(d) Rückgewinnen der am Ende des Schrittes (c) erhaltenen Nanopartikel.

16. Mesoporöse Nanopartikel, die durch ein Verfahren nach Anspruch 15 erhalten wurden.

17. Kit zum Detektieren einer Erkrankung ausgewählt aus Krebs, Tumoren, proliferativen zellulären Krankheiten, enthaltend:
- Nanopartikel nach den Ansprüchen 1 bis 10 oder eine Medikamentenzusammensetzung, die sie enthält, und
- Mittel, die eine LED-Bestrahlung, Blau- bis NIR-Laserbestrahlung ermöglichen.

## Claims

1. Mesoporous organosilica nanoparticles formed by elements comprising or constituted of :
(i) a porphyrin derivative selected from among :
- a compound having the formula A 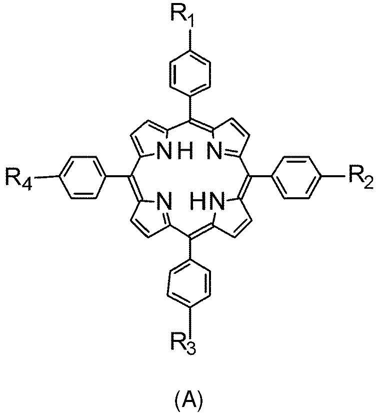 in which:
- Either R₁, R₂, R₃, and R₄ all correspond to 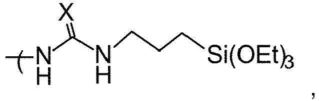 where X is the oxygen atom or the sulfur atom ;
- Or R₁, R₂, R₃, and R₄ all correspond to 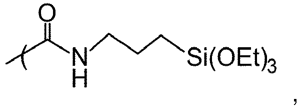
- a compound having the formula B, C or D, in which Z is selected from the oxygen atom or the sulfur atom 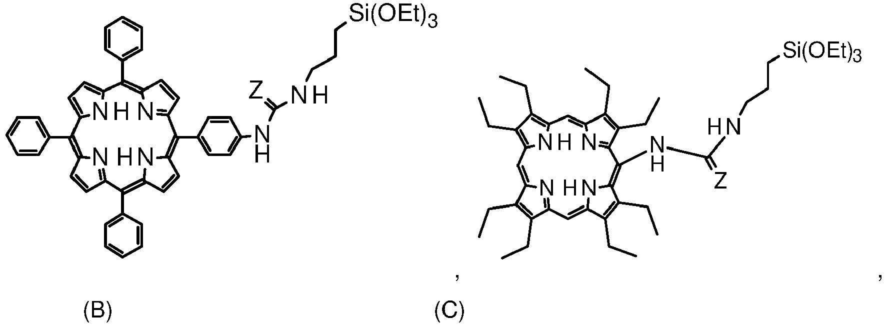 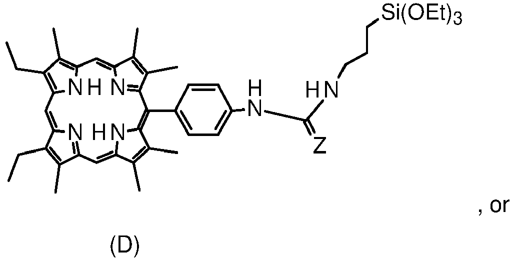
- a compound E, F or G 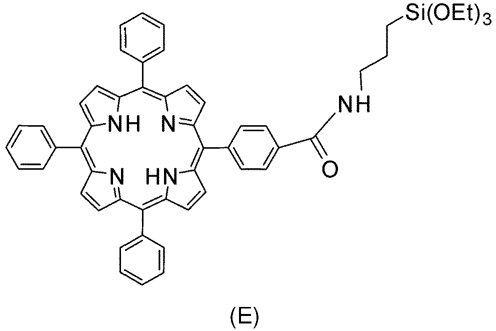 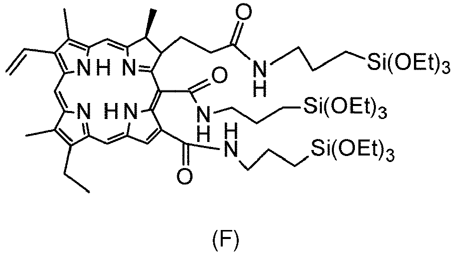 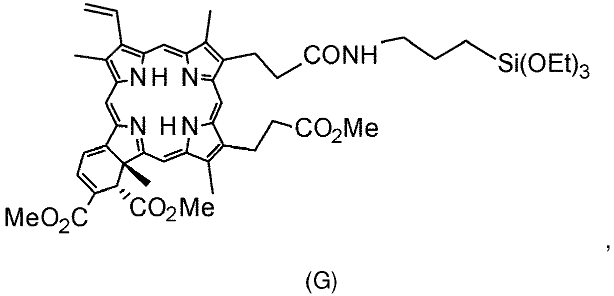 and
(ii) a compound having the formula I :
(EtO)₃Si(CH₂)ₙSi(OEt)₃,
in which n represents an integer selected from 1 to 10,
and possibly
(iii) a compound having the formula II
(EtO)₃Si(CH₂)₃-(S-S)ₘ-(CH₂)₃-Si(OEt)₃
in which m is an integer that is equal to 2 or 4,
said nanoparticles possibly encapsulating at least one hydrophilic and/or hydrophobic anticancer compound.

2. Nanoparticles according to claim 1, in which said hydrophilic anticancer compound is selected from gemcitabine, gemcitabine monophosphate, 5-fluorouracil, cytarabine, topotecane, irinotecane, or oxalylplatin; said hydrophobic anticancer compound is selected from doxorubicin, paclitaxel, or camptothecin.

3. Nanoparticles according to claim 1 or 2, in which the porphyrin derivative is a compound having the formula A and aggregates into an aggregate.

4. Nanoparticles according to claim 3, formed by the elements constituted of :
(i) a porphyrin derivative as defined in claim 1, in particular a porphyrin derivative having the formula here below: 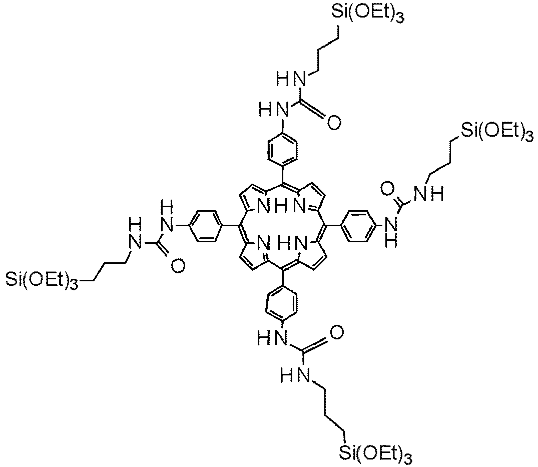 or a porphyrin derivative having the formula B, and
(ii) a compound having the formula I as defined in to claim 1.

5. Nanoparticles according to claim 3, formed by the elements constituted of :
(i) a porphyrin derivative as defined in claim 1, in particular a porphyrin derivative having the formula here below 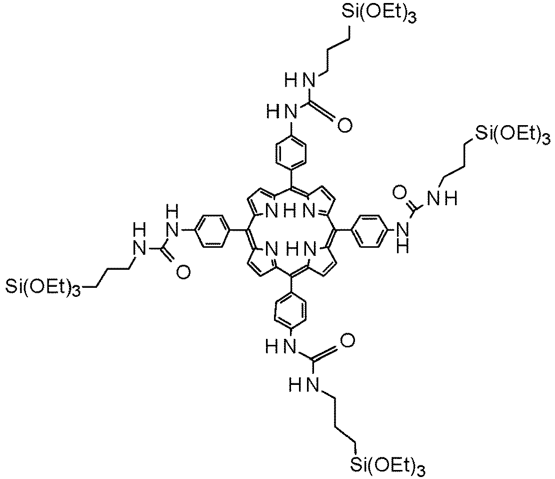 or a porphyrin derivative having the formula B, and
(ii) a compound having the formula I as defined in claim 1,
said nanoparticles encapsulating gemcitabine or gemcitabine monophosphate.

6. Nanoparticles according to claim 3, formed by the elements constituted of :
(i) a porphyrin derivative as defined in claim 1, in particular a porphyrin derivative having the formula here below 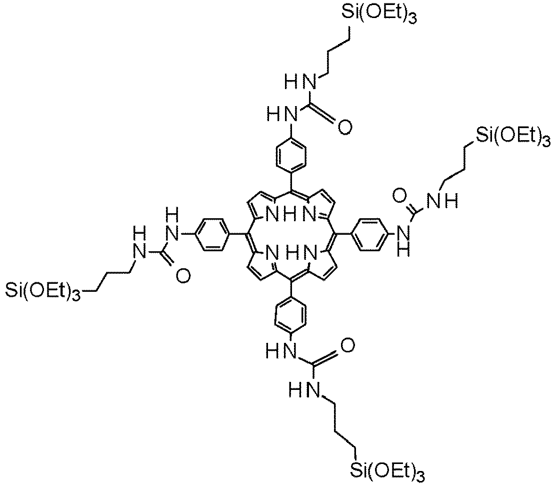 or a porphyrin derivative having the formula B,
(ii) a compound having the formula I as defined in to claim 1,
(iii) a compound having the formula II as defined in to claim 1.

7. Nanoparticles according to claim 3, formed by the elements constituted of:
(i) a porphyrin derivative as defined in claim 1, in particular a porphyrin derivative having the formula 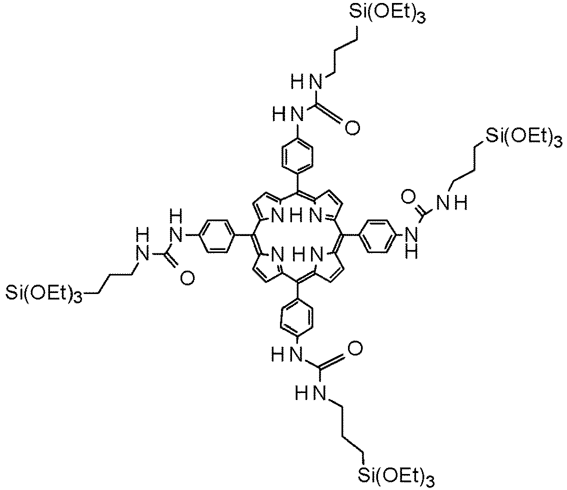 or a porphyrin derivative having the formula B,
(ii) a compound having the formula I as defined in to claim 1,
(iii) a compound having the formula II as defined in claim 1,
said nanoparticles encapsulating gemcitabine or gemcitabine monophosphate.

8. Nanoparticles according to any one of claims 1 to 7, in which the molar ratio between the porphyrin derivative and the compound having the formula I is between 2:98 and 20:80.

9. Nanoparticles according to any one of claims 1 to 3, 5 and 7, in which the load of hydrophilic or hydrophobic anticancer compound as defined in claim 1 is from 2% to 100% by weight, in particular 40% by weight, relative to the initial weight of the nanoparticles prior to the encapsulation of said anticancer compound.

10. Nanoparticles according to any one of claims 1 to 9, wherein the diameter of particles is from 20 to 400 nm, advantageously from 50 to 300 nm, more advantageously from 200 to 250 nm ; the specific area is from 100 to 1500 m²/g, advantageously from 800 to 1000 m²/g.

11. Nanoparticles according to any one of claims 1 to 10, as a medicament in the treatment of cancers, tumours, cell proliferative disorders and diseases, or skin diseases.

12. Nanoparticles according to any one of claims 1 to 10, for their use as a luminescent agent or fluorescent agent after irradiation in the detection or monitoring by imaging, in particular by means of two-photon microscopy, of cancers, tumours, cell proliferative disorders and diseases, or skin diseases.

13. Nanoparticles according to any one of claims 1 to 10, for their use as a photosensitising agent.

14. Pharmaceutical composition comprising the nanoparticles as claimed in any one of claims 1 to 10 and a pharmaceutically acceptable carrier.

15. Method for preparing the nanoparticles according to any one of claims 1 to 10, said method comprises the steps of :
(a) reacting in a basic aqueous solution at a temperature from 50°C to 90°C in the presence of a surfactant, the compounds comprising :
(i) a porphyrin derivative selected from among :
- a compound having the formula A 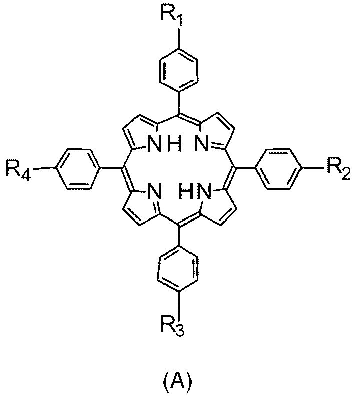 in which:
- Either R₁, R₂, R₃, and R₄ all correspond to 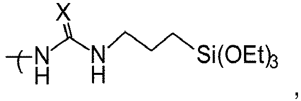 where X is the oxygen atom or the sulfur atom;
- Or R₁, R₂, R₃, and R₄ all correspond to 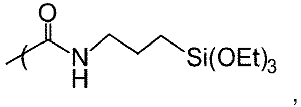
- a compound having the formula B, C or D, in which Z is selected from the oxygen atom or the sulfur atom 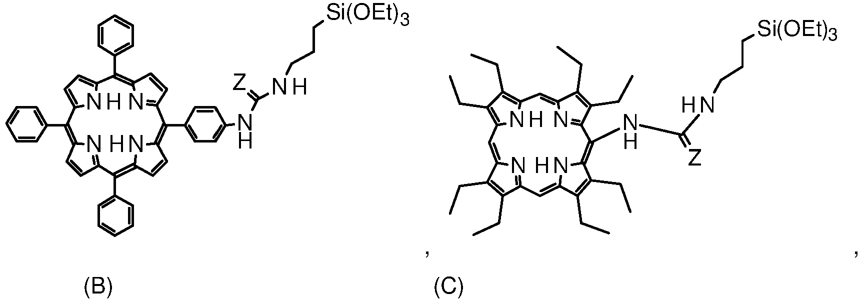 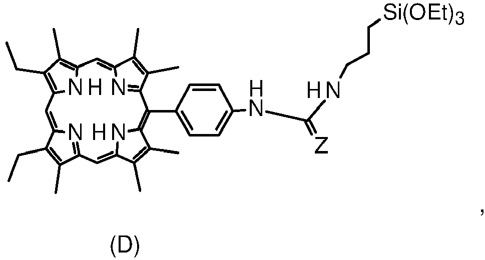 or
- a compound E, F or G 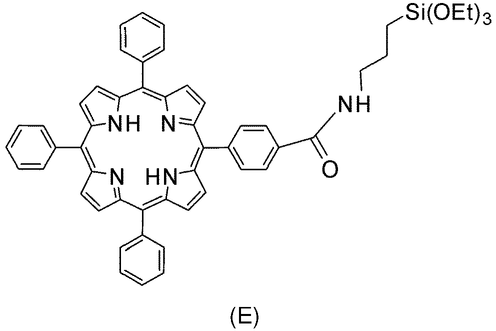 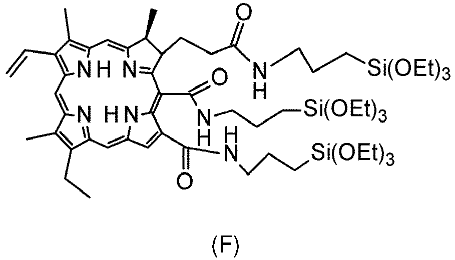 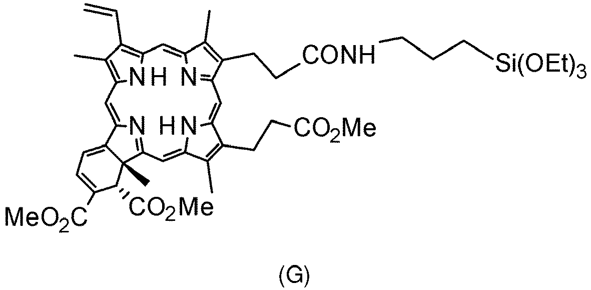
(ii) a compound having the formula I here below
(EtO)₃Si(CH₂)ₙSi(OEt)₃,
in which n represents an integer selected from 1 to 10,
and possibly
(iii) a compound having the formula II
(EtO)₃Si(CH₂)₃-(S-S)ₘ-(CH₂)₃-Si(OEt)₃
in which m is an integer that is equal to 2 or 4, and
(b) recovering the nanoparticles formed in the preceding step, and
possibly,
(c) reacting in a solvent the nanoparticles obtained in the step (b) with at least one hydrophilic and/or hydrophobic anticancer compound in order to encapsulate the latter,
(d) recovering the nanoparticles obtained at the end of the step (c).

16. Mesoporous nanoparticles obtained by the method according to claim 15.

17. A kit for the detection of a pathology selected from among cancers, tumours and cell proliferative disorders and diseases, the kit comprising :
- nanoparticles according to claim 1 to 10 or a medicine composition comprising the same; and
- means that provide for LED, blue to near IR laser irradiation.
